# EUROPEAN PATENT APPLICATION

(11) **EP 2 848 615 A1**
(43) Date of publication of application: **18.03.2015**
(21) Application number: 13382265.0
(22) Date of filing: 03.07.2013
(51) Int. Cl.: C07D 403/12, C07D 401/14, C07D 231/14, C07D 231/40, C07D 401/12, C07D 471/04, A61K 31/415, A61K 31/4155, A61P 29/00, A61P 37/00, A61P 35/00

(54) **New pyrazole derivatives as CRAC channel modulators**

(71) Applicant: Almirall, S.A., 08022 Barcelona (ES)
(72) Inventor: GONZALEZ RODRIGUEZ, JACOB, 08980 Barcelona (ES); VIDAL JUAN, BERNAT, 08980 Barcelona (ES); GUAL ROIG, SILVIA, 08980 Barcelona (ES)
(74) Representative: Elzaburu Marquez, Alberto

(57) **Abstract**

The present invention relates to compounds of formula (I) which are inhibitors of CRAC channel activity. This invention also relates to pharmaceutical compositions containing them, process for their preparation and their use in therapy.

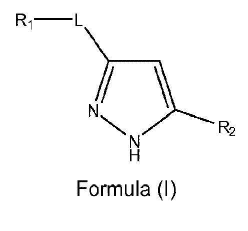

## Description

### FIELD OF THE INVENTION

The present invention relates to novel compounds which are inhibitors of CRAC channel activity. This invention also relates to pharmaceutical compositions containing them, process for their preparation and their use in therapy.

### BACKGROUND OF THE INVENTION

The regulation of intracellular calcium is a key element in the transduction of signals into and within cells. Cellular responses to growth factors, neurotransmitters, hormones and other signal molecules are initiated through Calcium-dependent processes. Virtually all cell types depend upon the generation of cytoplasmic calcium signals to regulate cell function or to trigger specific responses. Cytosolic calcium signals control a wide array of cellular functions ranging from short-term responses such as contraction and secretion to longer-term regulation of cell growth and proliferation. These signals involve combination of Calcium release from intracellular stores such as the endoplasmic reticulum (ER), and influx of Calcium across the plasma membrane.

In immune cells the molecular mechanisms of calcium signalling are especially well characterized. Cell activation through immunoreceptor engagement induces a rise in cytosolic calcium levels mainly through a selective channel-based process known as Store-Operated Calcium Entry (SOCE). Calcium Release-activated calcium (CRAC) channels are a type of SOC channels present in B and T-lymphocytes, NK cells, macrophages, DC and mast cells among the immune cells. CRAC channels are characterized by an extremely high ion selectivity for Ca²⁺ and a low conductance, and are activated through the Ca²⁺ sensors proteins in the ER called stromal interaction molecule (STIM) that bind to the pore forming unit of the CRAC channel in the plasma membrane, called ORAI. Upon antigen binding to the T-cell receptor, the activation of phospholipase C (PLCγ) generates the lipid metabolite InsP3, which promotes the release of Ca²⁺ from the ER stores. STIM proteins sense the calcium depletion in the ER, oligomerize and redistribute into discrete puncta located in junctional ER sites in close proximity to the plasma membrane. STIM proteins directly interact with ORAI1 in the plasma membrane opening the CRAC channel and allowing a sustained calcium entry across the plasma membrane (see Oh-hora, M. Immuno. Rev. 2009, 231, 210; Vig, M. Science 2006, 312, 1220; Vig, M. Nature Immunol. 2008, 9 (1), 89). Such a prolonged intracellular calcium increase activates short term processes such as degranulation, and cytosolic signal transduction processes involving gene transcription through NFAT to produce lipid mediators, several cytokines Th1, Th2, and Th17, matrix metalloproteinases, all of which participate in the pathogenesis of autoimmune and inflammation-based diseases.

Therefore, the involvement of deregulated lymphocyte Ca²⁺ signaling in the pathogenesis of autoimmune and inflammatory immune disorders suggests that interference with Ca²⁺ signaling through the CRAC channel may be a useful approach to treat autoimmune diseases, such as SLE or rheumatoid arthritis, and inflammatory disorders, such as respiratory diseases, psoriasis or ulcerative colitis, and to prevent allograft rejection in transplantation (Feske, S. Nature Reviews 2007, 7, 690).

More specifically, impaired CRAC channel activity has been linked to a growing number of diseases, including asthma, allergic diseases, autoimmunity, polyposis, inflammatory bowel disease and certain cancers. Therefore, CRAC channel inhibitors can be of considerable clinical benefit (Parekh, A. B. Nat. Rev. Drug Discov. 2010, 9, 399; Feske, S. Ann. N. Y. Acad. Sci. 2011, 1238, 74).

Patents and patent applications related to modulation of CRAC channel include WO2005009539, WO2006034402, WO2006081389, WO2007087427, WO2008039520, WO2009017818, WO2009035818 WO2009038775, WO20120025295, WO20100034003, WO20100039236, WO2010122088, WO2010122089, WO2011139489, WO2012027710, WO2012064808, WO2012052458, WO2012052459, WO2012079020, WO2012151355. Patent application WO2011036130 discloses indole derivatives as CRAC modulators.

While progress has been made in this field, there remains the need to identify further compounds which are CRAC channel modulators.

The present invention relates to novel bicyclic derivatives which are inhibitors of CRAC channel activity. Such derivatives have potential therapeutic benefit in the treatment of disorders associated with inappropriate CRAC activity, in particular in the treatment and prevention of allergic, inflammatory and autoimmune disorders including asthma, chronic obstructive pulmonary disease, bronchiectasis, cystic fibrosis, nasal polyposis (Di Capite J. L. Curr. Opin. Allergy Clin. Immunol. 2011, 11, 33), allergic rhinitis (Wang, Y. Exp. Mol. Med. 2012, 44 (3), 177), cough (Sutovska, M., Advances in Experimental Medicine and Biology, Chapter 6, DOI 10.1007/978-94-007-4549-0_6), allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, myeloproliferative diseases, chronic lymphocytic leaukaemia, B cell lymphoma, breast and prostate cancer, immune thrombocytopenic purpura, macular degeneration (Shaw, P. Frontiers in Bioscience E4 2012, 2253; Parekh, A. B. Nat. Rev. Drug Discov. 2010, 9, 399).

In view of the numerous conditions that are contemplated to benefit by treatment involving the inhibition of calcium release-activated calcium channel (CRAC), it is immediately apparent that new compounds that inhibit CRAC pathways and use of these compounds should provide substantial therapeutic benefits to a wide variety of patients.

It has now been found that bicyclic heteroaryl derivatives are novel and potent CRAC ORAI1 inhibitors and can therefore be used in the treatment or prevention of these diseases.

### SUMMARY OF THE INVENTION

Thus, the present invention is directed to compounds of formula (I), or pharmaceutically acceptable salts, N-oxides, or isotopically-labeled derivates thereof. 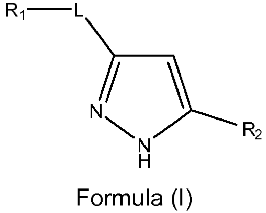 wherein:
L is selected from the group consisting of -CO-NH-, -NH-CO- and -NH-CH₂- group, wherein in the case of -NH-CH₂- group, the -CH₂- moiety is attached to the pyrazolyl ring while the -NH- moiety is attached to the R₁ substituent,
R₁ is selected from the group consisting of: 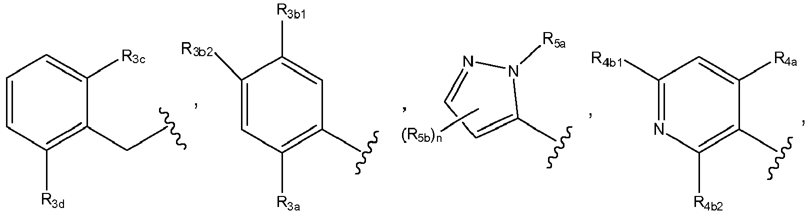 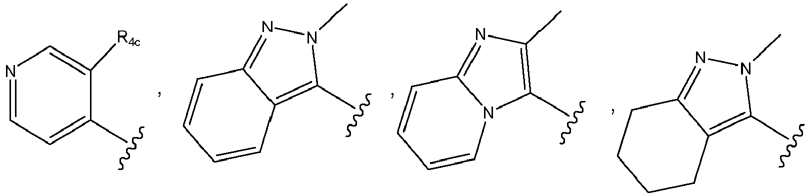 wherein
   R₃ₐ is independently selected from the group consisting of a halogen atom, a-COOH group, a C₁₋₄ alkyl group and a C₁₋₄ alkoxy group,
   R_{3b1} and R_{3b2} independently are selected from the group consisting of a hydrogen atom, a halogen atom, a C₁₋₄ alkyl group, a C₁₋₄ alkoxy group, a -CO-NR'R" group and a -SO₂-NR'R" group, wherein R' and R" are independently selected from the group consisting of a hydrogen atom, and a C₁₋₄ alkyl group, R_{3c} and R_{3d} are independently selected from the group consisting of halogen atom, C₁₋₄ alkyl group and C₁₋₅ alkoxy group,
   R₄ₐ is selected from the group consisting of a hydrogen atom, a halogen atom, a C₁₋₄ alkyl group and a C₁₋₄ alkoxy group,
   R_{4b1} and R_{4b2} independently are selected from the group consisting of a hydrogen atom, a C₁₋₄ alkyl group and a C₁₋₄ alkoxy group,
   R_{4c} is selected from the group consisting of a halogen atom, a C₁₋₄ alkyl group and a C₁₋₄ alkoxy group
   R₅ₐ represents a C₁₋₄ alkyl group or a C₃₋₆ cycloalkyl group, wherein the alkyl and the cycloalkyl groups are optionally substituted with one or more fluorine atoms.
   R_{5b} is independently selected from the group consisting of a C₁₋₄ alkyl group, a C₃₋₆ cycloalkyl group, a C₅₋₈ aryl group and a 5-to 8-membered heteroaryl group containing at least one heteroatom selected from N, S and O, wherein the alkyl
   group is optionally substituted with one or more fluorine atom,
R₂ is selected from the group consisting of: 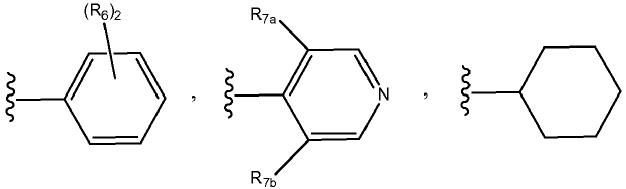 Wherein:
   R₆ is selected from the group consisting of a halogen atom, a C₁₋₄ alkyl group, and a C₁₋₄ alkoxy group,
   R₇ₐ and R_{7b} independently represent a hydrogen atom, a halogen atom or a C₁₋₂ alkyl group, with the proviso that both R₇ₐ and R_{7b} cannot be a hydrogen atom,
n has a value of 1, 2 or 3,
wherein the compound of formula (I) is not 2-chloro-5-(N,N-diethylsulfamoyl)-N-(5-(2,6-difluorophenyl)-1 H-pyrazol-3-yl)benzamide, N-(5-(5-bromo-2-chlorophenyl)-1H-pyrazol-3-yl)-2-fluorobenzamide nor N-(5-(2,6-difluorophenyl)-1H-pyrazol-3-yl)-1,3-dimethyl-1H-pyrazole-5-carboxamide.

The invention also provides synthetic processes and intermediates described herein, which are useful for preparing compounds of the invention.

The invention further provides a pharmaceutical composition comprising at least a compound of the invention and a pharmaceutically acceptable carrier.

The invention also provides a compound of the invention as described herein for use in the treatment of human or animal body by therapy.

The invention is also directed to the compounds as described herein, for use in the treatment of a pathological condition or disease associated with CRAC activity in particular wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease.

The invention also provides the use of the compounds of the invention as described herein, for the manufacture of a medicament for the treatment of a pathological condition or disease associated with CRAC activity, in particular wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease.

The invention is also directed to a method of treatment of a pathological condition or disease associated with CRAC activity, in particular wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, comprising administering a therapeutically effective amount of the compounds of the invention or a pharmaceutical composition of the invention to a subject in need of such treatment.

The invention also provides a combination product comprising (i) at least a compound of the invention as described herein; and (ii) one or more therapeutic active ingredients for simultaneous, separate or sequential use in the treatment of the human or animal body.

### DETAILED DESCRIPTION OF THE INVENTION.

When describing the compounds, compositions and methods of the invention, the following terms have the following meanings, unless otherwise indicated.

As used herein the term C₁-C₄ alkyl embraces linear or branched radicals having 1 to 4 carbon atoms. Examples include methyl, ethyl, n-propyl, i-propyl, n-butyl, sec-butyl and t-butyl radicals.

As used herein, the term C₁-C₅ alkoxy (or alkyloxy) embraces optionally substituted, linear or branched oxy-containing radicals each having alkyl portions of 1 to 5 carbon atoms. Examples include methoxy, ethoxy, n-propoxy, i-propoxy, n-butoxy, sec-butoxy, t-butoxy and pentoxy radicals.

As used herein, the term C₆-C₈ aryl radical embraces typically a C₆-C₅, preferably a C₆-C₈ aryl radical such as phenyl group.

As used herein, the term 5- to 8- membered heteroaryl radical embraces typically a 5-to 8- membered ring system comprising at least one heteroaromatic ring and containing at least one heteroatom selected from O, S and N. Examples include pyridyl, pyrazinyl, pyrimidinyl, pyridazinyl, furyl, oxadiazolyl, oxazolyl, isoxazolyl, benzoxazolyl, imidazolyl, benzimidazolyl, thiazolyl, thiadiazolyl, thienyl, pyrrolyl, and benzothiazolyl.

As used herein, the term C₃-C₆ cycloalkyl radical embraces saturated monocyclic or polycyclic carbocyclic radicals having from 3 to 6 carbon atoms. Examples of cycloalkyl groups include cyclopropyl, cyclobutyl, cyclopentyl and cyclohexyl.

As used herein, the term halogen atom embraces chlorine, fluorine, bromine and iodine atoms. A halogen atom is typically a fluorine, chlorine or bromine atom, most preferably chlorine or fluorine. The term halo when used as a prefix has the same meaning.

Also included within the scope of the invention are the isomers, polymorphs, pharmaceutically acceptable salts, N-oxides, isotopes, solvates and prodrugs of the compounds of formula (I). Any reference to a compound of formula (I) throughout the present specification includes a reference to any isomer, polymorph, pharmaceutically acceptable salt, N-oxide, isotope, solvate or prodrug of such compound of formula (I).

### Isomers

Compounds containing one or more chiral centre may be used in enantiomerically or diastereoisomerically pure form, in the form of racemic mixtures and in the form of mixtures enriched in one or more stereoisomer. The compounds of Formula (I) as described and claimed encompass the racemic forms of the compounds as well as the individual enantiomers, diastereomers, and stereoisomer-enriched mixtures.

Conventional techniques for the preparation/isolation of individual enantiomers include chiral synthesis from a suitable optically pure precursor or resolution of the racemate using, for example, chiral high pressure liquid chromatography (HPLC). Alternatively, the racemate (or a racemic precursor) may be reacted with a suitable optically active compound, for example, an alcohol, or, in the case where the compound contains an acidic or basic moiety, an acid or base such as tartaric acid or 1-phenylethylamine. The resulting diastereoisomeric mixture may be separated by chromatography and/or fractional crystallization and one or both of the diastereoisomers converted to the corresponding pure enantiomer(s) by means well known to one skilled in the art. Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% isopropanol, typically from 2 to 20%, and from 0 to 5% of an alkylamine, typically 0.1 % diethylamine. Concentration of the eluate affords the enriched mixture. Stereoisomer conglomerates may be separated by conventional techniques known to those skilled in the art. See, e.g. "Stereochemistry of Organic Compounds" by Ernest L. Eliel (Wiley, New York, 1994).

Atropisomers are stereoisomers resulting from hindered rotation about single bonds where the steric strain barrier to rotation is high enough to allow for the isolation of the conformers. Oki (Oki, M; Topics in Stereochemistry 1983, 1) defined atropisomers as conformers that interconvert with a half-life of more than 1000 seconds at a given temperature. The scope of the invention as described and claimed encompasses the racemic forms of the compounds as well as the individual atropisomers (an atropisomer "substantially free" of its corresponding enantiomer) and stereoisomer-enriched mixtures, i.e. mixtures of atropisomers.

Separation of atropisomers is possibly by chiral resolution methods such as selective crystallization. In an atropo-enantioselective or atroposelective synthesis one atropisomer is formed at the expense of the other. Atroposelective synthesis may be carried out by use of chiral auxiliaries like a Corey-Bakshi-Shibata (CBS) catalyst (asymmetric catalyst derived from proline) in the total synthesis of knipholone or by approaches based on thermodynamic equilibration when an isomerization reaction favors one atropisomer over the other.

The compounds of Formula (I) may exhibit the phenomena of tautomerism and structural isomerism. Tautomers exist as mixtures of a tautomeric set in solution. In solid form, usually one tautomer predominates. Even though one tautomer may be described, the present invention includes all tautomers of the compounds of Formula (I).

### Polymorphs

The compounds of formula (I) may exist in different physical forms, i.e. amorphous and crystalline forms.

Moreover, the compounds of the invention may have the ability to crystallize in more than one form, a characteristic which is known as polymorphism. Polymorphs can be distinguished by various physical properties well known in the art such as X-ray diffraction pattern, melting point or solubility. All physical forms of the compounds of formula (I), including all polymorphic forms ("polymorphs") thereof, are included within the scope of the invention.

### Pharmaceutically acceptable salts

As used herein, the term pharmaceutically acceptable salt refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid; and organic acids, for example citric, fumaric, gluconic, glutamic, lactic, maleic, malic, mandelic, mucic, ascorbic, oxalic, pantothenic, succinic, tartaric, benzoic, acetic, methanesulphonic, ethanesulphonic, benzenesulphonic, p-toluenesulphonic acid, xinafoic (1-hydroxy-2-naphthoic acid), napadisilic (1,5-naphthalenedisulfonic acid) and the like. Particularly preferred are salts derived from fumaric, hydrobromic, hydrochloric, acetic, sulfuric, methanesulfonic, xinafoic, and tartaric acids.

Salts derived from pharmaceutically-acceptable inorganic bases include aluminum, ammonium, calcium, copper, ferric, ferrous, lithium, magnesium, manganic, manganous, potassium, sodium, zinc and the like. Particularly preferred are ammonium, calcium, magnesium, potassium and sodium salts.

Salts derived from pharmaceutically-acceptable organic bases include salts of primary, secondary and tertiary amines, including alkyl amines, arylalkyl amines, heterocyclyl amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

Other preferred salts according to the invention are quaternary ammonium compounds wherein an equivalent of an anion (X⁻) is associated with the positive charge of the N atom. X⁻ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X⁻ is preferably an anion selected from chloride, bromide, iodide, sulphate, nitrate, acetate, maleate, oxalate, succinate or trifluoroacetate. More preferably X⁻ is chloride, bromide, trifluoroacetate or methanesulphonate.

### N-oxides

As used herein, an N-oxide is formed from the tertiary basic amines or imines present in the molecule, using a convenient oxidising agent.

### Isotopes

The invention also includes isotopically-labeled derivatives of the compounds of the invention, wherein one or more atoms is replaced by an atom having the same atomic number, but an atomic mass or mass number different from the atomic mass or mass number usually found in nature. Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I , nitrogen, such as ¹³N and ¹⁵N, oxygen, such as ¹⁵O, ¹⁷O and ¹⁸O, phosphorus, such as ³²P, and sulfur, such as ³⁵S. Certain isotopically-labeled compounds of the invention, for example, those incorporating a radioactive isotope, are useful in drug and/or substrate tissue distribution studies. The radioactive isotopes tritium, ³H, and carbon-14, ¹⁴C, are particularly useful for this purpose in view of their ease of incorporation and ready means of detection. Substitution with heavier isotopes such as deuterium, ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled derivatives of the compounds of the invention can generally be prepared by conventional techniques known to those skilled in the art or by processes analogous to those described herein, using an appropriate isotopically-labeled reagent in place of the non-labeled reagent otherwise employed.

Preferred isotopically-labeled derivatives include deuterated derivatives of the compounds of the invention. As used herein, the term deuterated derivative embraces compounds of the invention where in a particular position at least one hydrogen atom is replaced by deuterium. Deuterium (D or ²H) is a stable isotope of hydrogen which is present at a natural abundance of 0.015 molar %.

### Solvates

The compounds of the invention may exist in both unsolvated and solvated forms. The term solvate is used herein to describe a molecular complex comprising a compound of the invention and an amount of one or more pharmaceutically acceptable solvent molecules. The term hydrate is employed when said solvent is water. Examples of solvate forms include, but are not limited to, compounds of the invention in association with water, acetone, dichloromethane, 2-propanol, ethanol, methanol, dimethylsulfoxide (DMSO), ethyl acetate, acetic acid, ethanolamine, or mixtures thereof. It is specifically contemplated that in the present invention one solvent molecule can be associated with one molecule of the compounds of the present invention, such as a hydrate.

Furthermore, it is specifically contemplated that in the present invention, more than one solvent molecule may be associated with one molecule of the compounds of the present invention, such as a dihydrate. Additionally, it is specifically contemplated that in the present invention less than one solvent molecule may be associated with one molecule of the compounds of the present invention, such as a hemihydrate. Furthermore, solvates of the present invention are contemplated as solvates of compounds of the present invention that retain the biological effectiveness of the non-solvate form of the compounds.

### Prodrugs

Prodrugs of the compounds described herein are also within the scope of the invention. Thus certain derivatives of the compounds of the present invention, which derivatives may have little or no pharmacological activity themselves, when administered into or onto the body may be converted into compounds of the present invention having the desired activity, for example, by hydrolytic cleavage. Such derivatives are referred to as 'prodrugs'. Further information on the use of prodrugs may be found in Pro-drugs as Novel Delivery Systems, Vol. 14, ACS Symposium Series (T. Higuchi and W. Stella) and Bioreversible Carriers in Drug Design, Pergamon Press, 1987 (ed. E. B. Roche, American Pharmaceutical Association).

Prodrugs in accordance with the invention can, for example, be produced by replacing appropriate functionalities present in the compounds of the present invention with certain moieties known to those skilled in the art as 'pro-moieties' as described, for example, in Design of Prodrugs by H. Bundgaard (Elsevier, 1985).

Typically, in the compound of formula (I), L is selected from the group consisting of-CO-NH-, -NH-CO-, preferably, -NH-CO-, wherein the -CO- moiety is attached to the pyrazolyl ring while the -NH- moiety is attached to the R₁ substituent.

Typically, in the compound of formula (I), R₁ is selected from the group consisting of: 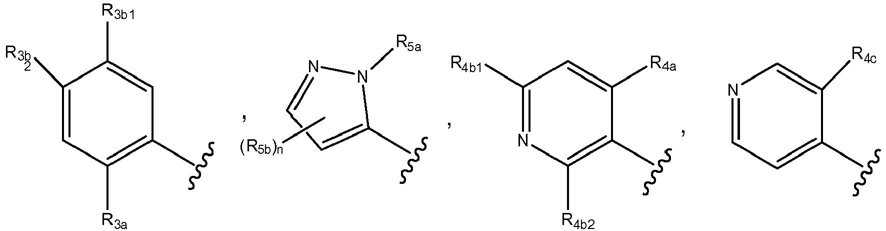 wherein R₃ₐ, R_{3b1}, R_{3b2}, R₄ₐ, R_{4b1}, R_{3b2}, R_{4c}, R₅ₐ, R_{5b} and n are as defined above.

In a preferred embodiment, R₁ is selected from the group consisting of: 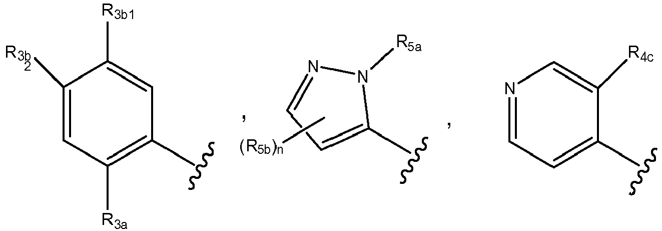 wherein R₃ₐ, R_{3b1}, R_{3b2}, R_{4c}, R₅ₐ, R_{5b} and n are as defined above.

In a still more preferred embodiment, R₁ is selected from the group consisting of: 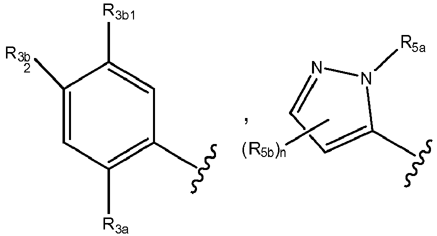 wherein R₃ₐ, R_{3b1}, R_{3b2}, R₅ₐ, R_{5b} and n are as defined above.

Typically, in the compound of formula (I):
(a) R₃ₐ represents a halogen atom or a C₁₋₄ alkoxy group, preferably a C₁₋₄ alkoxy group, more preferably a metoxy group, and/or
(b) R_{3b1} and R_{3b2} independently are selected from the group consisting of a hydrogen atom, a halogen atom, a C₁₋₄ alkoxy group, a -CO-NR'R" group and a -SO₂-NR'R" group, wherein R' and R" are independently selected from the group consisting of a hydrogen atom, and a methyl group, preferably R_{3b1} is selected from the group consisting of a methoxy group, a -CO-NR'R" group and a -SO₂-NR'R" group, wherein both R' and R" represents a methyl group while R_{3b2} represents a hydrogen atom, more preferably, R_{3b1} represents a methoxy group while R_{3b2} represents a hydrogen atom,.

Typically, in the compound of formula (I), both R_{3c} and R_{3d} represent a halogen atom or a methoxy group, preferably both R_{3c} and R_{3d} represent a chlorine atom.

Typically, in the compound of formula (I):
(a) R₄ₐ is selected from the group consisting of a hydrogen atom and a C₁₋₄ alkyl group, preferably a hydrogen atom or a methyl group, and/or
(b) R_{4b1} and R_{4b2} independently are selected from the group consisting of a hydrogen atom, a methyl group and a methoxy group, preferably R_{4b1} represents a hydrogen atom or a methyl group while R_{4b2} represents a methoxy group.

Typically, in the compound of formula (I):
(a) R₅ₐ represents an non-substituted C₁₋₃ alkyl group, preferably a methyl or an ethyl group, and/or
(b) R_{5b} is selected from the group consisting of a C₁₋₄ alkyl group and a C₅₋₈ aryl group, wherein the alkyl group is optionally substituted with one or more fluorine atom, preferably, R_{5b} represents a -CF₃ group or a phenyl group, wherein preferably n has a value of 1 or 2, more preferably 1.

Typically, in the compound of formula (I), R₂ is selected from the group consisting of: 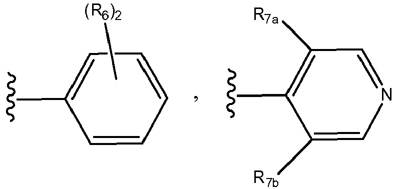 wherein R₆, R₇ₐ and R_{7b} are as defined above, preferably, both R₇ₐ represents a halogen atom and R_{7b} a hydrogen atom, more preferably, R₇ₐ represents a fluorine atom. In a preferred embodiment, R₂ represents a phenyl group substituted with two substituents selected from halogen atom and methoxy group, preferably halogen atoms, more preferably chlorine and fluorine atoms.

Typically, in the compound of formula (I):
R₃ₐ is independently selected from the group consisting of a -COOH group, a methyl group and a methoxy group,
R_{3b1} is selected from the group consisting of a hydrogen atom, methoxy group, a -CO-NR'R" group and a -SO₂-NR'R" group, wherein R' and R" are independently selected from the group consisting of a hydrogen atom and a methyl group,
R_{3b2} represents a hydrogen atom,
Both R_{3c} and R_{3d} represent a chlorine atom,
R₄ₐ is selected from the group consisting of a hydrogen atom and a methyl group,
R_{4b1} is selected from the group consisting of a hydrogen atom and a methyl group,
R_{4b2} represents a metyoxy group,
R_{4c} represents a fluorine atom,
R₅ₐ represents a methyl or an ethyl group,
R_{5b} is selected from the group consisting of a methyl group, -CF₃ group, a phenyl group and a pyridyl group,
R₆ is selected from the group consisting of a fluorine atom, a chlorine atom and a methoxy group,
R₇ₐ represents a hydrogen atom and R_{7b} represents a fluorine atom,
n has a value of 1 or 2,

Typically, in the compound of formula (I):
L represents -NH-CO- group, wherein the -CO- moiety is attached to the pyrazolyl ring while the -NH- moiety is attached to the R₁ substituent R₁ represents 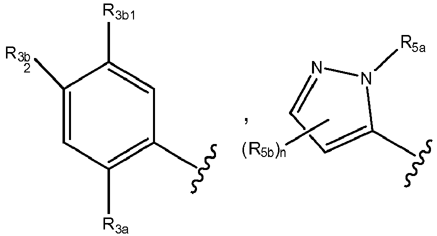 wherein
   R₃ₐ represents a methoxy group,
   R_{3b}, represents a methoxy group while R_{3b2} a hydrogen atom,
   R₅ₐ represents a methyl or an ethyl group,
   R_{5b} represents a -CF₃ group or a phenyl group.
R₂ represents a phenyl group substituted with two substituents selected from chlorine and fluorine atoms.

Particular individual compounds of the present invention include:
5-(2-chloro-6-fluorophenyl)-*N*-[1-methyl-3-(trifluoromethyl)-1*H*-pyrazol-5-yl]-1*H-*pyrazole-3-carboxamide
*N*-[5-(2-chloro-6-fluorophenyl)-1*H*-pyrazol-3-yl]-1-methyl-3-(trifluoromethyl)-1*H-*pyrazole-5-carboxamide
5-(2-chloro-6-fluorophenyl)-*N*-(6-methoxy-4-methylpyridin-3-yl)-1*H*-pyrazole-3-carboxamide
5-(2-chloro-6-fluorophenyl)-*N*-(2,6-dichlorobenzyl)-1*H*-pyrazole-3-carboxamide
5-(2-chloro-6-fluorophenyl)-*N*-(2,5-dimethoxyphenyl)-1*H*-pyrazole-3-carboxamide
5-(2-chloro-6-fluorophenyl)-*N*-(1-methyl-3-phenyl-1*H*-pyrazol-5-yl)-1*H*-pyrazole-3-carboxamide
5-(2-chloro-6-fluorophenyl)-*N*-(6-methoxy-2-methylpyridin-3-yl)-1*H*-pyrazole-3-carboxamide
5-(2,5-dimethoxyphenyl)-*N*-(3-fluoropyridin-4-yl)-1*H*-pyrazole-3-carboxamide
5-(2,5-dimethoxyphenyl)-*N*-[1-methyl-3-(trifluoromethyl)-1*H*-pyrazol-5-yl]-1*H*-pyrazole-3-carboxamide
*N*-[5-(2,5-dimethoxyphenyl)-1*H*-pyrazol-3-yl]-3-fluoroisonicotinamide
5-(2-chloro-6-fluorophenyl)-*N*-(3-fluoropyridin-4-yl)-1*H*-pyrazole-3-carboxamide
*N*-[5-(2-chloro-6-fluorophenyl)-1*H*-pyrazol-3-yl]-2,5-dimethoxybenzamide
*N*-[5-(2,5-dimethoxyphenyl)-1*H*-pyrazol-3-yl]-1-methyl-3-(trifluoromethyl)-1*H*-pyrazole-5-carboxamide
5-(2-chloro-6-fluorophenyl)-*N*-(2-methylimidazo[1,2-a]pyridin-3-yl)-1*H*-pyrazole-3-carboxamide
5-cyclohexyl-*N*-(1-methyl-3-(trifluoromethyl)-1*H*-pyrazol-5-yl)-1*H*-pyrazole-3-carboxamide
5-(2-chloro-6-fluorophenyl)-*N*-(2-methoxy-5-(methylcarbamoyl)phenyl)-1*H*-pyrazole-3-carboxamide
5-(2-chloro-6-fluorophenyl)-*N*-(5-(dimethylcarbamoyl)-2-methoxyphenyl)-1H-pyrazole-3-carboxamide
5-(2-chloro-6-fluorophenyl)-*N*-(2-methyl-5-sulfamoylphenyl)-1H-pyrazole-3-carboxamide
5-(2-chloro-6-fluorophenyl)-N-(5-(N,N-dimethylsulfamoyl)-2-methoxyphenyl)-1H-pyrazole-3-carboxamide
5-(3-fluoropyridin-4-yl)-N-(1-methyl-3-(trifluoromethyl)-1H-pyrazol-5-yl)-1H-pyrazole-3-carboxamide
2-(5-(2-chloro-6-fluorophenyl)-1H-pyrazole-3-carboxamido)benzoic acid
N-(5-carbamoyl-2-methoxyphenyl)-5-(2-chloro-6-fluorophenyl)-1H-pyrazole-3-carboxamide
5-(2-chloro-6-fluorophenyl)-N-(1,3-dimethyl-1H-pyrazol-5-yl)-1H-pyrazole-3-carboxamide
5-(2-chloro-6-fluorophenyl)-*N*-[1-ethyl-3-(trifluoromethyl)-1*H*-pyrazol-5-yl]-1*H*-pyrazole-3-carboxamide
5-(2-chloro-6-fluorophenyl)-*N*-(2-methyl-2*H*-indazol-3-yl)-1*H-*pyrazole-3-carboxamide
5-(2-chloro-6-fluorophenyl)-*N*-(1-methyl-3-pyridin-4-yl-1*H*-pyrazol-5-yl)-1*H*-pyrazole-3-carboxamide
5-(2-chloro-6-fluorophenyl)-*N*-(1,4-dimethyl-3-phenyl-1*H*-pyrazol-5-yl)-1*H*-pyrazole-3-carboxamide
*N*-{[5-(2-chloro-6-fluorophenyl)-1*H*-pyrazol-3-yl]methyl}-1-methyl-3-(trifluoromethyl)-1 H-pyrazol-5-amine
5-(2-chloro-6-fluorophenyl)-N-(2-methyl-4,5,6,7-tetrahydro-2H-indazol-3-yl)-1H-pyrazole-3-carboxamide
and pharmaceutically acceptable salts, N-oxides and isotopically-labeled derivates thereof.

Of outstanding interest are:
5-(2-chloro-6-fluorophenyl)-*N*-[1-methyl-3-(trifluoromethyl)-1*H*-pyrazol-5-yl]-1*H-*pyrazole-3-carboxamide
*N*-[5-(2-chloro-6-fluorophenyl)-1*H*-pyrazol-3-yl]-1-methyl-3-(trifluoromethyl)-1*H-*pyrazole-5-carboxamide
5-(2-chloro-6-fluorophenyl)-*N*-(2,5-dimethoxyphenyl)-1*H*-pyrazole-3-carboxamide
5-(2-chloro-6-fluorophenyl)-*N*-(1-methyl-3-phenyl-1*H*-pyrazol-5-yl)-1*H*-pyrazole-3-carboxamide
*N*-[5-(2,5-dimethoxyphenyl)-1*H*-pyrazol-3-yl]-3-fluoroisonicotinamide
*N*-[5-(2-chloro-6-fluorophenyl)-1*H*-pyrazol-3-yl]-2,5-dimethoxybenzamide
*N*-[5-(2,5-dimethoxyphenyl)-1*H*-pyrazol-3-yl]-1-methyl-3-(trifluoromethyl)-1*H*-pyrazole-5-carboxamide
5-(2-chloro-6-fluorophenyl)-N-(5-(dimethylcarbamoyl)-2-methoxyphenyl)-1H-pyrazole-3-carboxamide
5-(2-chloro-6-fluorophenyl)-N-(5-(N,N-dimethylsulfamoyl)-2-methoxyphenyl)-1H-pyrazole-3-carboxamide
5-(2-chloro-6-fluorophenyl)-*N*-[1-ethyl-3-(trifluoromethyl)-1*H*-pyrazol-5-yl]-1*H*-pyrazole-3-carboxamide
5-(2-ch!oro-6-fluorophenyl)-*N*-(1-methyl-3-pyridin-4-yl-1*H*-pyrazol-5-yl)-1*H*-pyrazole-3-carboxamide
5-(2-chloro-6-fluorophenyl)-N-(2-methyl-4,5,6,7-tetrahydro-2H-indazol-3-yl)-1H-pyrazole-3-carboxamide
and pharmaceutically acceptable salts, N-oxides and and isotopically-labeled derivates thereof.

The invention also provides a compound of the invention as described herein for use in the treatment of human or animal body by therapy.

The invention is also directed to the compounds as described herein, for use in the treatment of a pathological condition or disease associated with CRAC activity in particular wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease.

The invention also provides the use of the compounds of the invention as described herein, for the manufacture of a medicament for the treatment of a pathological condition or disease associated with CRAC activity, in particular wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease.

The invention is also directed to a method of treatment of a pathological condition or disease associated with CRAC activity, in particular wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, comprising administering a therapeutically effective amount of the compounds of the invention or a pharmaceutical composition of the invention to a subject in need of such treatment.

The invention also provides a combination product comprising (i) at least a compound of the invention as described herein; and (ii) one or more other therapeutic active ingredients, for simultaneous, separate or sequential use in the treatment of the human or animal body.

As used herein, the term therapeutically effective amount refers to an amount sufficient to effect treatment when administered to a patient in need of treatment.

As used herein, the term treatment refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

As used herein, the term disease or condition associated with CRAC activity includes all disease states and/or conditions that are acknowledged now, or that are found in the future, to be associated with CRAC activity. Such disease states include, but are not limited to, an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease.

### GENERAL SYNTHETIC PROCEDURES

The compounds of the invention can be prepared using the methods and procedures described herein, or using similar methods and procedures. It will be appreciated that where typical or preferred process conditions (i.e., reaction temperatures, times, mole ratios of reactants, solvents, pressures, etc.) are given; other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group, as well as suitable conditions for protection and deprotection, are well known in the art. For example, numerous protecting groups, and their introduction and removal are described in T. W. Greene and G. M. Wuts, Protecting Groups in Organic Synthesis, Third Edition, Wiley, New York, 1999, and references cited therein.

Processes for preparing compounds of the invention are provided as further embodiments of the invention and are illustrated by the procedures below.

Compounds of general formula (Ib), i.e. compounds of formula (I) wherein L represents -NH-CH2-, may be prepared following the synthetic scheme depicted in Scheme 1.

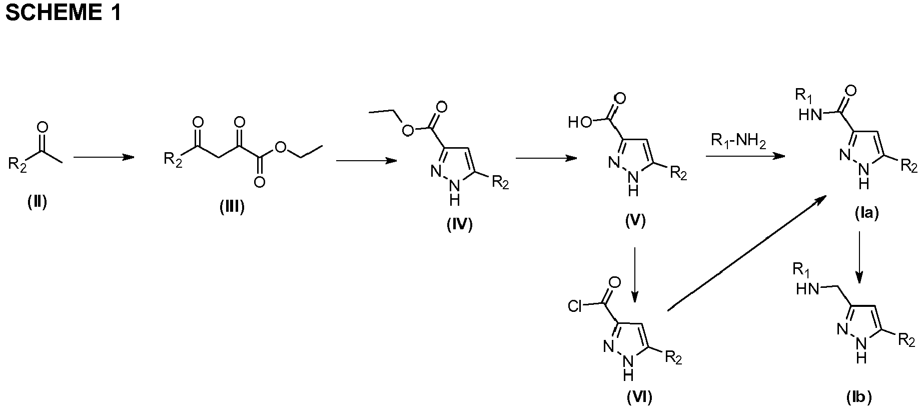

Reaction of an appropriate methyl ketone (II) with diethyl oxalate in the presence of a base such as sodium hydride in an aprotic organic solvent as toluene at a temperature of 90°C provides compounds of general formula (III). Cyclization of compounds (III) with hydrazine hydrate under acidic conditions (for instance using acetic acid as a solvent) at 120°C provides intermediates of general formula (IV). Hydrolysis in a basic conditions (for instance a bases such as sodium hydroxide) and using ethanol as a solvent at room temperature provides key intermediates (V). These compounds can be converted into compounds of general formula (Ia) (i.e. compounds of formula(I), wherein L represents -CO-NH-) by reaction with appropriate amines R₁-NH₂ in the presence of N-(3-dimethylaminopropyl)-N'-ethylcarbodiimide hydrochloride and 1-hydroxybenzotriazole hydrate in a aprotic organic solvent such as DMF at room temperature.

Alternatively, compounds of general formula (Ib) can be prepared by chlorination of compounds of general formula (V) with thionyl chloride in a solvent such as toluene at 110°C to provide the intermediates (VI) which undergo further reaction with the amines R₁-NH₂ in the presence of a base such as triethylamine and in a solvent such as dichloromethane at room temperature.

Reduction of compounds of formula (Ia) ((i.e. compounds of formula(I), wherein L represents -CO-NH-) with borane tetrahydrofuran complex in tetrahydrofuran as a solvent at 50°C provides the compounds of formula (Ib).

Compounds of general formula (Ic), i.e. compounds of formula (I) wherein L represents -NH-CO-, can be prepared as illustrated in scheme 2.

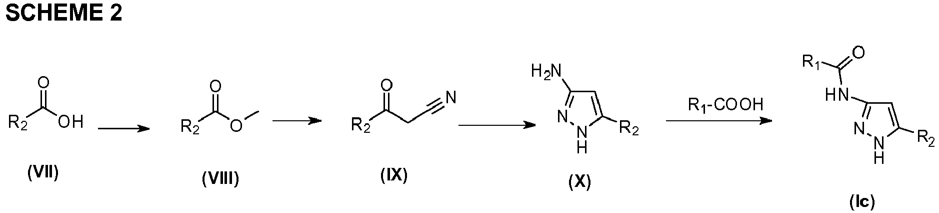

Esterification of compound of formula (VII) in methanol under acidic conditions (for example in the presence of sulfuric acid) provides compounds of formula (VIII). Further reaction of (VIII) with acetonitrile in a solvent that is inert to the reaction conditions such as tetrahydrofuran, in the presence of organolithium base such as lithium diisopropylamide at -78°C provides intermediates of formula (IX). Such compounds can be converted into compounds of formula (X) by cyclization with hydrazine hydrate in ethanol under microwave irradiation at 120°C. Such compounds can be converted into compounds of general formula (Ic) by reaction with acids R₁-COOH in the presence of N-(3-dimethylaminopropyl)-N'-ethylcarbodiimide and 1-hydroxybenzotriazole hydrate in a aprotic organic solvent such as DMF.

### EXAMPLES

The syntheses of the compounds of the invention are illustrated by the following Examples (1 to 29) including Preparations (1 to 25) which do not limit the scope of the invention in any way.

### General

Reagents, starting materials, and solvents were purchased from commercial suppliers and used as received. Concentration or evaporation refers to evaporation under vacuum using a Büchi rotatory evaporator.

Reaction products were purified, when necessary, by flash chromatography on silica gel (40-63 µm) with the solvent system indicated. Purifications in reverse phase were made in a Biotage SP1^{®} automated purification system equipped with a C₁₈ column and using a gradient of water/acetonitrile/MeOH (1:1) (0.1% v/v ammonium formate both phases) from 0% to 100% acetonitrile/MeOH (1:1) in 40 column volumes. The appropriate fractions were collected and the solvents evaporated under reduced pressure and/or liofilized.

Preparative HPLC-MS were performed on a Waters instrument equipped with a 2767 injector/collector, a 2525 binary gradient pump, a 2996 PDA detector, a 515 pump as a make-up pump and a ZQ4000 Mass spectrometer detector.

The chromatographic separations were obtained using a Waters 2795 system equipped with a Symmetry C₁₈ (2.1 x 50 mm, 3.5 µm) column for methods A , B and C and a Symmetry C₁₈ (2.1 x 100 mm, 3.5 µm) for method D. The mobile phase was formic acid (0.4 ml), ammonia (0.1 ml), methanol (500 ml) and acetonitrile (500 ml) (B) and formic acid (0.5 ml), ammonia (0.125 ml) and water (1000 ml) (A), the gradients are specified in the following table for each method used.

| Method | Run Time | 0% B | 0 to 95% B | 95% B |
|---|---|---|---|---|
| A | 5 min | 0.2 min | 3 min | 0.8 min |
| B | 9 min | 0.5 min | 6.5 min | 1 min |
| C | 15 min | 0 min | 10.5 min | 1.5 min |

The flow rate was 0.8 ml/min for method A and 0.4 ml/min for method B, C and D. The injection volume was 5 microliter. A Waters 2996 diode array was used as a UV detector. Chromatograms were processed at 210 nM or 254 nM. Mass spectra of the chromatograms were acquired using positive and negative electrospray ionization in a Micromass ZMD or in a Waters ZQ detectors coupled to the HPLC.

¹H Nuclear Magnetic Resonance Spectra were recorded on a Varian Gemini-2000 spectrometer operating at a frequency of 300 MHz for the ¹H spectra or in a Varian Mercury plus operating at a frequency of 400 MHz for the ¹H spectra. Samples were dissolved in the specified deuterated solvent. Tetramethylsilane was used as reference.

The UPLC chromatographic separations were obtained using a Waters Acquity UPLC system coupled to a SQD mass spectrometer detector. The system was equipped with an ACQUITY UPLC BEH C-18 (2.1x50mm, 1.7 mm) column. The mobile phase was formic acid (0.4 ml), ammonia (0.1 ml), methanol (500 ml) and acetonitrile (500 ml) (B) and formic acid (0.5 ml), ammonia (0.125 ml) and water (1000 ml) (A). A gradient between 0 to 95% of B was used. The run time was 3 or 5 minutes The injection volume was 0.5 microliter. Chromatograms were processed at 210 nM or 254 nM. Mass spectra of the chromatograms were acquired using positive and negative electrospray ionization.

Mass Spectra (m/z) were recorded on a Micromass ZMD or in a Waters ZQ mass spectrometer using ESI ionization.

### Abbreviations:

- DMF: Dimethylformamide
- DMSO: Dimethylsulfoxide
- CDCl₃: Deuterated chloroform
- NMR: Nuclear magnetic resonance
- s: Singlet
- d: Doublet
- dd: Doublet doublet
- td: Triple doublet
- br: Broad
- q: Quartet
- t: Triplet
- m: Multiplet
- LRMS: Low resolution mass spectrometry
- h: hour
- min: minutes

### PREPARATION 1

### Ethyl 4-(2-chloro-6-fluorophenyl)-2,4-dioxobutanoate

To a solution of 1-(2-chloro-6-fluorophenyl)ethanone (1.00 g, 5.8 mmol) in anhydrous toluene (12 mL) was added portionwise NaH (60% in oil, 0.25 g, 6.2 mmol). After stirring for 1 h at rt a solution of diethyl oxalate (1.22 mL, 8.7 mmol) in toluene (5 mL) was added, and the mixture was heated at 90°C in a sealed tube overnight. Aqueous saturated NH₄Cl solution was added, and the mixture was extracted with ethyl acetate. The organic layer was washed with water, brine and dried over MgSO₄, filtered, and the solvents were removed under reduced pressure and the residue was purified using the Isolera purification (diethyl ether - hexane gradient, 0:100 rising to 100:0) to give 0.8 g (2.93 mmol, 50%) of the title compound as a brown oil.
LRMS: m/z 273 (M+1)⁺.

### PREPARATION 2

### Ethyl 5-(2-chloro-6-fluorophenyl)-1H-pyrazole-3-carboxylate

A solution of ethyl 4-(2-chloro-6-fluorophenyl)-2,4-dioxobutanoate (Preparation 1) (0.5 g, 1.83 mmol), acetic acid (2 mL) and hydrazine hydrate (100 µL, 2.0 mmol) was heated at 120°C in a sealed tube overnight. The reaction mixture was then poured into iced water, basified with saturated aqueous NaHCO₃ solution and the product was extracted with ethyl acetate. The ethyl acetate layer was collected and washed with water and brine solution, dried over MgSO₄ and concentrated under reduced pressure. The resulting residue was purified using the Isolera purification (diethyl ether - hexane gradient, 0:100 rising to 100:0) to give 0.285 g (1.1 mmol, 58%) of the title compound as a colorless oil.
LRMS: m/z 269 (M+1)⁺.

### PREPARATION 3

### 5-(2-chloro-6-fluorophenyl)-1H-pyrazole-3-carboxylic acid

Ethyl 5-(2-chloro-6-fluorophenyl)-1H-pyrazole-3-carboxylate (Preparation 2) (0.285mg, 1.06 mmol) was dissolved in EtOH (4 mL), 1.3 mL of NaOH 8.0 N were added and the mixture was stirred overnight at room temperature. Ethanol was evaporated, and the residue was dissolved in water, the aqueous layer is acidified with 6 N HCl and extracted four times with dichloromethane. The combined organic extracts was dried over MgSO₄, filtered, evaporated, and dried in vacuo to give 0.210mg (8.6 mmol, 81%) of the title compound as a white solid.
LRMS: m/z 241 (M+1)⁺.

### PREPARATION 4

### Ethyl 4-(2,5-dimethoxyphenyl)-2,4-dioxobutanoate

1-(2,5-dimethoxyphenyl)ethanone was treated with diethyl oxalate, NaH (60% in oil) and toluene a solvent according to the method described in Preparation 1 to obtain the title compound (93% yield).
LRMS (m/z): 281 (M+1)⁺.

### PREPARATION 5

### Ethyl 5-(2,5-dimethoxyphenyl)-1H-pyrazole-3-carboxylate

ethyl 4-(2,5-dimethoxyphenyl)-2,4-dioxobutanoate (Preparation 4) was treated with acetic acid and hydrazine hydrate according to the method described in Preparation 2 to obtain the title compound (85% yield).
LRMS (m/z): 277(M+1)⁺.

### PREPARATION 6

### 5-(2,5-dimethoxyphenyl)-1H-pyrazole-3-carboxylic acid

Ethyl 5-(2,5-dimethoxyphenyl)-1*H*-pyrazole-3-carboxylate (Preparation 5) was treated with NaOH 8.0 N and ethanol as a solvent according to the method described in Preparation 3 to obtain the title compound (95% yield).
LRMS (m/z): 249 (M+1)⁺.

### PREPARATION 7

### Methyl 2-chloro-6-fluorobenzoate

A mixture of 2-chloro-6-fluorolbenzoic acid (1.0 g, 5.7 mmol) in methanol (10 mL) and a few drops of concentrated sulfuric acid were added. The reactions took place in 3h under microwave irradiation at 110°C. After cooling to room temperature, methanol was evaporated, and the residue was dissolved in ethyl acetate. The solution was extracted with saturated NaHCO₃ .The organic layer was dried over MgSO₄, filtered, evaporated, and dried in vacuo, affording 0.64 g (3.39 mmol, 59%) of methyl 2-chloro-6-fluorolbenzoate. The product was used without further purification.

### PREPARATION 8

### 3-(2-chloro-6-fluorophenyl)-3-oxopropanenitrile

To a solution of diisopropyl amine (1.22 mL, 8.68 mmol) in THF (10 mL), under argon is added dropwise at -78°C a solution of butyl lithium (5.41 mL, 8.66 mmol, 1.6M in hexane) and the solution was stirred at -78°C for 30min. A solution of acetonitrile (0.30 mL, 5.7 mmol) in THF (5 mL) was added dropwise. After the mixture was stirred at - 78°C for 1h, methyl 2-chloro-6-fluorolbenzoate (Preparation 7) (1.1 g, 5.8 mmol) in 5 mL THF was added dropwise at -78°C. The reaction mixture was kept at -78°C for 1 h and warmed up to RT. After being stirred for an additional 1 h, the reaction is quenched by addition of H₂O. The aqueous layer is acidified to pH 5 with 2 N HCl and extracted two times with ethyl acetate. The combined organic extracts were washed with water, brine and dried over MgSO₄ and concentrated to give 1.07 g (5.4 mmol, 94%) of the title compound as a orange oil.
LRMS (m/z): 196 (M-1).

### PREPARATION 9

### 5-(2-chloro-6-fluorophenyl)-1H-pyrazol-3-amine

To a suspension of 3-(2-chloro-6-fluorophenyl)-3-oxopropanenitrile (Preparation 8) (0.6g, 3.04 mmol) in ethanol (3 mL), hydrazine monohydrate (0.2 mL, 5.24 mmol) was added and the reactions was heated for 3h under microwave irradiation at 120°C. The mixture was concentrated and the residue was dissolved in ethyl acetate. The organic phase was washed with water, brine and dried over MgSO₄ and evaporated under reduced pressure and the residue was purified using the Isolera purification system (methanol-dichloromethane, gradient, 0:100 rising to 2:98) to give 0.44 g (2.1mmol, 68%) of the title compound as a yellow solid.
LRMS (m/z): 212 (M+1)⁺.

### PREPARATION 10

### Methyl 2,5-dimethoxybenzoate

2,5-dimethoxybenzoic acid was treated with concentrated sulfuric acid and methanol as a solvent according to the method described in Preparation 7 to obtain the title compound (57% yield).
LRMS (m/z): 197 (M+1)⁺.

### PREPARATION 11

### 3-(2,5-dimethoxyphenyl)-3-oxopropanenitrile

Methyl 2,5-dimethoxybenzoate (Preparation 10) was treated with diisopropyl amine, butyl lithium (1.6M in hexane), acetonitrile and tetrahidrofurane as a solvent according to the method described in Preparation 8 to obtain the title compound (87% yield).
LRMS (m/z): 206 (M+1)⁺.

### PREPARATION 12

### 5-(2,5-dimethoxyphenyl)-1H-pyrazol-3-amine

3-(2,5-dimethoxyphenyl)-3-oxopropanenitrile (Preparation 11) was treated hydrazine monohydrate and ethanol as a solvent according to the method described in Preparation 9 to obtain the title compound (52% yield).
LRMS (m/z): 220 (M+1)⁺.

### PREPARATION 13

### 2-methyl-3-nitroimidazo[1,2-a]pyridine

To a cooled solution of 2-methylimidazo[1,2-a]pyridine (380 mg, 2.88 mmol) in concentated sulfuric acid (6 mL), was added nitric acid (472 µl, d = 1.38). After being stirred for 2 h at room temperature, the solution is crushed on ice, made basic by the addition of aqueous saturated Na₂CO₃ solution and extracted with CH₂Cl₂. The organic layers were dried in MgSO₄, filtered and evaporated to dryness to give 330 mg (1.82 mmol, 65%) of the title compound as a brown solid.
LRMS (m/z): 178(M+1)⁺.

### PREPARATION 14

### 2-methylimidazo[1,2-a]pyridin-3-amine

2-methyl-3-nitroimidazo[1,2-a]pyridine (Preparation 13) (330.0 mg, 1.86 mmol) was dissolved in 8 ml ethanol. Under argon atmosphere palladium on carbon (10%, 20 mg, 0.2 mmol) was added and the reaction mixture was hydrogenated for 18 h. The catalyst was filtered through a pad of Celite and the filtered was concentrated under reduced pressure to give 260 mg (1.76 mmol, 95% yield) of the title compound as a brown solid.
LRMS: m/z 148 (M+1)⁺.

### PREPARATION 15

### Ethyl 4-cyclohexyl-2,4-dioxobutanoate

A mixture of 1-cyclohexylethanone (6.5 g, 0.05 mol) and diethyl oxalate (7 mL, 0.05 mol) was added dropwise to a solution of sodium (1.32g, 0.06 mol) in ethanol (25 mL) maintaining the temperature below 5°C. After being stirred for 48h at room temperature the mixture was cooled to 0°C and sulfuric acid (20%) is added till pH 1-2. Then water was added and extracted twice with diethyl ether. The combined organic phase were washed with brine, dried over Na₂SO₄, filtered and concentrated to give 7.7 g ( 0.03 mol, 66%) of the title compound as a yellow oil.
LRMS (m/z): 227 (M+1)⁺.

### PREPARATION 16

### Ethyl 5-cyclohexyl-1H-pyrazole-3-carboxylate

To suspension of 4-cyclohexyl-2,4-dioxobutanoate (Preparation 15, 1g, 4.71 mmol) in ethanol (3 mL), hydrazine monohydrate (0.23 mL, 4.72 mmol) was added and the reaction was heated for 3h at 105°C under nitrogen atmosphere. Ethanol was evaporated and the residue was dissolved in ethyl acetate, washed with water, dried over Na₂SO₄, filtered and concentrated under reduced pressure. The resulting residue was purified using the Isolera purification (ethyl acetate-hexane 0:100 rising to 100:0) to give 574 mg (2.52 mmol, 53%) of the title compound as a white solid.
LRMS (m/z): 223 (M+1)⁺.

### PREPARATION 17

### 5-cyclohexyl-1 H-pyrazole-3-carboxylic acid

Ethyl 5-cyclohexyl-1H-pyrazole-3-carboxylate (Preparation 16) was treated with NaOH 8.0 N and ethanol as a solvent according to the method described in Preparation 3 to obtain the title compound (96% yield).
LRMS (m/z): 195 (M+1)⁺.

### PREPARATION 18

### 3-amino-4-methoxy-N-methylbenzamide

3-amino-4-methoxybenzoic acid (500 mg, 2.99 mmol) and methanamine (7.48 mL, 14.96 mmol) were dissolved in 5 mL DMF. N-[(dimethylamino)-1H-1,2,3-triazol[4,5-b]pyridin-1 (1.19 g, 3.14 mmol) and diisopropylethylamine (625 µL, 3.59 mmol) were added and the mixture was stirred at room temperature for 18 hours. Dichloromethane was added and the organic phase was washed with saturated aqueous NaHCO₃ solution, water and brine, dried over Na₂SO₄ and evaporated under reduced pressure and the residue was purified using the Isolera purification (ethyl acetate - hexane gradient, 0:100 rising to 100:0) to give 155 mg (0.86 mmol, 29%) of the title compound.
LRMS (m/z): 181 (M+1)⁺.

### PREPARATION 19

### 5-(2-chloro-6-fluorophenyl)-1H-pyrazole-3-carbonyl chloride

To a solution of compound of Preparation 3 ( 2 g, 8.3 mmol) in anhydrous toluene (20 mL) was added thionyl chloride (0.91 mL, 12.46 mmol) and the mixture was heated at 110°C for 2 hours under nitrogen athmosphere. The mixture was concentrated, toluene was added and the solution was evaporated under reduced pressure to obtain the title compound (2.07g, 93%).

### PREPARATION 20

### 3-amino-4-methoxy-N,N-dimethylbenzamide

3-amino-4-methoxybenzoic acid (500 mg, 2.99 mmol ) and dimethylamine 2M in THF (7.48 mL, 14.96 mmol) were dissolved in 5 mL DMF. N-[(dimethylamino)-1H-1,2,3-triazol[4,5-b]piridin-1 (1.19 g, 3.14 mmol) and diisopropylethylamine (625 µL, 3.59 mmol) were added and the mixture was stirred at room temperature for 18 hours. Dichloromethane was added and the organic phase was washed with saturated aqueous NaHCO₃ solution, water and brine, dried over Na₂SO₄ and evaporated under reduced pressure and the residue was purified using the Isolera purification (ethyl acetate - hexane gradient, 0:100 rising to 100:0) to give 570 mg (2.93 mmol, 98%) of the title compound.
LRMS (m/z): 195 (M+1)⁺.

### PREPARATION 21

### Ethyl 4-(3-fluoropyridin-4-yl)-2,4-dioxobutanoate

1-(3-fluoropyridin-4-yl)ethanone was treated with diethyl oxalate, NaH (60% in oil) and toluene a solvent according to the method described in Preparation 1 to obtain the title compound (30% yield).
LRMS (m/z): 240 (M+1)⁺.

### PREPARATION 22

### 5-(3-fluoropyridin-4-yl)-1H-pyrazole-3-carboxylic acid

A solution of ethyl 4-(3-fluoropyridin-4-yl)-2,4-dioxobutanoate (Preparation 21, 255 mg, 1.07 mmol), ethanol (2 mL) and hydrazine hydrate (50 µL, 1.07 mmol) was heated at 105°C in a sealed tube overnight. The reaction mixture was evaporated and hydrochloridric acid 37% (10 mL) was added. The solution was heated for 5 hours at 120°C in a sealed tube. The reaction mixture was then neutralized with NaOH 32% and purified by reverse phase chromatography to give the title compound.
LRMS (m/z): 208 (M+1)⁺.

### PREPARATION 23

### tert-butyl (2-methyl-2H-indazol-3-yl)carbamate

To a suspension of 2-methyl-2H-indazole-3-carboxylic acid (100 mg, 0.57 mmol) in toluene (1.5 mL) and *tert*-butanol (1.5 mL), triethylamine (0.24 mL, 1.72 mmol) and diphenylphosphoryl azide (0.37 mL, 1.72 mmol) were added and mixture was heated overnight at 100°C. Ethyl acetate was added and organic layer was washed with water and brine, dried over MgSO₄ and evaporated under reduced pressure. The residue was purified using the Isolera purification system (ethyl acetate - hexane gradient, 0:100 rising to 100:0) to give 90 mg (0.36 mmol, 98%) of the title compound.
LRMS (m/z): 248 (M+1)⁺.

### PREPARATION 24

### 2-methyl-2H-indazol-3-amine

To a solution of *tert*-butyl (2-methyl-2H-indazol-3-yl)carbamate (Preparation 23, 90 mg, 0.36 mmol) in dioxane (1 mL), 4N HCl in dioxane (2 mL, 8 mmol) was added and reaction was stirred at room temperature for 8 hours. Solvent was removed under reduced pressure to give the title compound as hydrochloride salt (66 mg, 0.36 mmol, 100%).
LRMS (m/z): 148 (M+1)⁺.

### PREPARATION 25

### 1-methyl-3-pyridin-4-yl-1H-pyrazol-5-amine

3-bromo-1-methyl-1H-pyrazol-5-amine (200 mg, 1.14 mmol) and 4-(4,4,5,5-tetramethyl-1,3,2-dioxaborolan-2-yl)pyridine (256 mg, 1.25 mmol) in dioxane (4.5 mL) and water (1.5 mL) were placed in a microwave vessel. Tris(dibenzylideneacetone)dipalladium(0) (52 mg, 0.06 mmol), tri-*tert*-butyl phosphonium tetrafluoroborate (33 mg, 0.11 mmol) and potassium phosphate (482 mg, 2.3 mmol) were then added under argon atmosphere and the mixture was subjected to microwave irradiation for 30 minutes at 120°C. Reaction mixture was filtered through a Celite pad and it was washed with ethyl acetate and water. The filtrate was concentrated under reduced pressure and the resulting crude was purified using the Isolera purification system (methanol - water gradient, 0:100 rising to 100:0) to give 118 mg (0.68 mmol, 60%) of the title compound.
LRMS (m/z): 370 (M+1)⁺.

### EXAMPLES

### EXAMPLE 1

### 5-(2-chloro-6-fluorophenyl)-N-[1-methyl-3-(trifluoromethyl)-1H-pyrazol-5-yl]-1H-pyrazole-3-carboxamide

Compound of Preparation 3 (100 mg, 0.42 mmol) and 1-methyl-3-(trifluoromethyl)-1H-pyrazol-5-amine (76 mg, 0.46 mmol) were dissolved in 1.5 mL DMF. N-(3-Dimethylaminopropyl)-N'-ethylcarbodiimide hydrochloride 98% (62 mg, 0.46 mmol) and 1-Hydroxybenzotriazole hydrate (88 mg, 0.46 mmol) were added and the mixture was stirred at room temperature for 7 days. Dichloromethane was added and the organic phase was washed with water two times, saturated aqueous NaHCO₃ solution and brine, dried over MgSO₄ and evaporated under reduced pressure and the residue was purified using the Isolera purification (diethyl ether - hexane gradient, 0:100 rising to 100:0) to give 54 mg (0.13 mmol, 33%) of the title compound as a white solid.
LRMS (m/z): 388 (M+1)⁺.
1 H NMR (300 MHz, CHLOROFORM-*d*) d ppm 3.90 (3 H, s), 6.69 (1 H, s), 7.11 - 7.23 (1 H, m), 7.33 - 7.45 (2 H, m), 8.69 (1 H, br. s)

### EXAMPLE 2

### N-[5-(2-chloro-6-fluorophenyl)-1H-pyrazol-3-yl]-1-methyl-3-(trifluoromethyl)-1H-pyrazole-5-carboxamide

Compound of Preparation 11 (100 mg, 0.19 mmol) and 1-methyl-3-(trifluoromethyl)-1 H-pyrazole-5-carboxylic acid (91 mg, 0.47 mmol) were dissolved in 1.5 mL DMF. N-(3-Dimethylaminopropyl)-N'-ethylcarbodiimide hydrochloride 98% (144 mg, 0.75 mmol) and 1-Hydroxybenzotriazole hydrate (102 mg, 0.75 mmol) were added and the mixture was stirred at room temperature overnight. Ethyl acetate was added and the organic phase was washed with water two times, NaOH 1 N two times, dried over MgSO₄ and evaporated under reduced pressure and the residue was purified using the Isolera purification (ethyl acetate - hexane gradient, 0:100 rising to 100:0) to give 35 mg (0.09 mmol, 19%) of the title compound as a white solid.
LRMS (m/z): 388 (M+1)⁺.
1 H NMR (300 MHz, DMSO-*d*₆) d ppm 4.19 (3 H, s), 6.86 (1 H, s), 7.31 - 7.61 (3 H, m), 7.65 (1 H, s), 11.24 (1 H, br. s), 12.97 (1 H, br. s)

### EXAMPLE 3

### 5-(2-chloro-6-fluorophenyl)-N-(6-methoxy-4-methylpyridin-3-yl)-1H-pyrazole-3-carboxamide

Obtained (43% yield) as a solid from 5-(2-chloro-6-fluorophenyl)-1H-pyrazole-3-carboxylic acid (Preparation 3) and 6-methoxy-4-methylpyridin-3-amine following the experimental procedure as described in Example 1.
LRMS (m/z): 361 (M+1)⁺.
1 H NMR (300 MHz, CHLOROFORM-*d*) d ppm 2.31 (3 H, s), 3.92 (3 H, s), 6.64 (1 H, s), 7.10 - 7.19 (1 H, m), 7.31 - 7.37 (3 H, m), 8.47 (1 H, br. s), 8.50 (1 H, s)

### EXAMPLE 4

### 5-(2-chloro-6-fluorophenyl)-N-(2,6-dichlorobenzyl)-1H-pyrazole-3-carboxamide

Obtained (51% yield) as a white solid from 5-(2-chloro-6-fluorophenyl)-1H-pyrazole-3-carboxylic acid (Preparation 3) and (2,6-dichlorophenyl) methanamine following the experimental procedure as described in Example 1.
LRMS (m/z): 400 (M+1)⁺.
1 H NMR (300 MHz, CHLOROFORM-*d*) d ppm 4.98 (2 H, d, J=5.87 Hz), 7.08 - 7.23 (4 H, m), 7.30 - 7.37 (4 H, m)

### EXAMPLE 5

### 5-(2-chloro-6-fluorophenyl)-N-(2,5-dimethoxyphenyl)-1H-pyrazole-3-carboxamide

Obtained (48% yield) as a solid from 5-(2-chloro-6-fluorophenyl)-1H-pyrazole-3-carboxylic acid (Preparation 3) and 2,5-dimethoxyaniline following the experimental procedure as described in Example 1.
LRMS (m/z): 376 (M+1)⁺.
1 H NMR (300 MHz, DMSO-*d*₆) d ppm 3.73 (3 H, s), 3.88 (3 H, s), 6.59 - 6.74 (1 H, m, J=6.61 Hz), 6.96 - 7.11 (2 H, m), 7.37 - 7.51 (1 H, m), 7.51 - 7.67 (2 H, m), 8.03 (1 H, s), 9.41 (1 H, , br. s), 13.87 (1 H, br. s)

### EXAMPLE 6

### 5-(2-chloro-6-fluorophenyl)-N-(1-methyl-3-phenyl-1H-pyrazol-5-yl)-1H-pyrazole-3-carboxamide

Obtained (22% yield) as a pale yellow solid from 5-(2-chloro-6-fluorophenyl)-1H-pyrazole-3-carboxylic acid (Preparation 3) and 1-methyl-3-phenyl-1 H-pyrazol-5-amine following the experimental procedure as described in Example 1.
LRMS (m/z): 396 (M+1)⁺.
1 H NMR (300 MHz, CHLOROFORM-*d*) d ppm 3.90 (3 H, s), 6.74 (1 H, s), 7.12 - 7.21 (1 H, m), 7.28 - 7.44 (5 H, m), 7.79 (2 H, d, J=7.04 Hz), 8.66 (1 H, s)

### EXAMPLE 7

### 5-(2-chloro-6-fluorophenyl)-N-(6-methoxy-2-methylpyridin-3-yl)-1H-pyrazole-3-carboxamide

Obtained (28% yield) as a yellow solid from 5-(2-chloro-6-fluorophenyl)-1H-pyrazole-3-carboxylic acid (Preparation 3) and 6-methoxy-2-methylpyridin-3-amine following the experimental procedure as described in Example 1.
LRMS (m/z): 361 (M+1)⁺. 1 H NMR (300 MHz, DMSO-*d*₆) d ppm 2.35 (3 H, s), 3.85 (3 H, s), 6.69 (1 H, d,
J=8.80 Hz), 6.97 (1 H, s), 7.33 - 7.78 (4 H, m), 9.80 (1 H, br. s), 13.79 (1 H, br. s)

### EXAMPLE 8

### 5-(2,5-dimethoxyphenyl)-N-(3-fluoropyridin-4-yl)-1H-pyrazole-3-carboxamide

Obtained (2% yield) as a yellow solid from 5-(2,5-dimethoxyphenyl)-1*H*-pyrazole-3-carboxylic acid (Preparation 3) and 3-fluoroisonicotinic acid following the experimental procedure as described in Example 1.
LRMS (m/z): 343 (M+1)⁺.
¹H NMR (300 MHz, DMSO-*d*₆) d ppm 3.79 (3 H, m), 3.87 (3 H, m), 6.98 (1 H, dd, J=8.80, 2.93 Hz), 7.12 (1 H, d, J=9.39 Hz), 7.31 (1 H, s), 7.38 (1 H, d, J=2.93 Hz), 8.11 - 8.26 (1 H, m), 8.40 (1 H, d, J=5.28 Hz), 8.60 (1 H, s), 9.78 (1 H, br. s), 13.67 (1 H, br. s)

### EXAMPLE 9

### 5-(2,5-dimethoxyphenyl)-N-[1-methyl-3-(trifluoromethyl)-1H-pyrazol-5-yl]-1H-pyrazole-3-carboxamide

Obtained (9% yield) as a yellow solid from 5-(2,5-dimethoxyphenyl)-1*H*-pyrazole-3-carboxylic acid acid (Preparation 3) and 1-methyl-3-(trifluoromethyl)-1H-pyrazol-5-amine following the experimental procedure as described in Example 1.
LRMS (m/z): 396 (M+1)⁺.
1 H NMR (300 MHz, DMSO-*d*₆) d ppm 3.73 - 3.83 (6 H, m,), 3.87 (3 H, s), 6.70 (1 H, s), 6.98 (1 H, dd, J=9.10, 2.35 Hz), 7.12 (1 H, d, J=9.10Hz), 7.24 (1 H, s), 7.36 (1 H, d, J=2.35 Hz), 10.38 (1 H, br. s), 13.63 (1 H, br. s)

### EXAMPLE 10

### N-[5-(2,5-dimethoxyphenyl)-1H-pyrazol-3-yl]-3-fluoroisonicotinamide

Obtained (27% yield) as a yellow solid from 5-(2,5-dimethoxyphenyl)-1*H*-pyrazol-3-amine (Preparation 11) and 3-fluoroisonicotinic acid following the experimental procedure as described in Example 2.
LRMS (m/z): 343 (M+1)⁺.
¹H NMR (300 MHz, DMSO-*d*₆) d ppm 3.64 (3 H, m), 3.77 (3 H, m), 5.93 (1 H, m), 6.76 - 6.84 (2 H, m), 6.90 - 6.99 (1 H, m), 7.02 (1 H, s), 7.03 - 7.08 (1 H, m), 7.75 - 7.85 (1 H, m), 8.59 - 8.67 (1 H, m), 8.80 (1 H, s)

### EXAMPLE 11

### 5-(2-chloro-6-fluorophenyl)-N-(3-fluoropyridin-4-yl)-1H-pyrazole-3-carboxamide

Obtained (10% yield) as a solid from 5-(2-chloro-6-fluorophenyl)-1H-pyrazole-3-carboxylic acid (Preparation 3) and 3-fluoropyridin-4-amine following the experimental procedure as described in Example 1.
LRMS (m/z): 335 (M+1)⁺.
¹H NMR (300 MHz, DMSO-*d*₆) d ppm 7.10 (1 H, s), 7.29 - 7.71 (4 H, m), 8.08 (1 H, s), 8.41 (1 H, d, J=5.28 Hz), 8.61 (1 H, d, J=2.35 Hz), 9.94 (1 H, br. s)

### EXAMPLE 12

### N-[5-(2-chloro-6-fluorophenyl)-1H-pyrazol-3-yl]-2,5-dimethoxybenzamide

Obtained (10% yield) as a white solid from 5-(2,5-dimethoxyphenyl)-1*H*-pyrazol-3-amine (Preparation 11) and 2,5-dimethoxybenzoic acid following the experimental procedure as described in Example 2.
LRMS (m/z): 376 (M+1)⁺.
¹H NMR (300 MHz, DMSO-*d*₆) d ppm 3.77 (3 H, s), 3.93 (3 H, s), 6.87 (1 H, s), 7.11 -7.20 (2 H, m), 7.34 - 7.41 (2 H, m), 7.49 - 7.58 (2 H, m), 10.46 (1 H, br. s), 12.85 (1 H, br. s)

### EXAMPLE 13

### N-[5-(2,5-dimethoxyphenyl)-1H-pyrazol-3-yl]-1-methyl-3-(trifluoromethyl)-1H-pyrazole-5-carboxamide

Obtained (51 % yield) as a white solid from 5-(2,5-dimethoxyphenyl)-1*H*-pyrazol-3-amine (Preparation 11) and 1-methyl-3-(trifluoromethyl)-1H-pyrazole-5-carboxylic acid following the experimental procedure as described in Example 2.
LRMS (m/z): 396 (M+1)⁺.
¹H NMR (300 MHz, DMSO-*d*₆) d ppm 3.78 (3 H, s), 3.85 (3 H, s), 4.20 (3 H, s), 6.87 - 6.97 (1 H, m,), 7.04 - 7.14 (2 H, m,), 7.27 (1 H, d, J=3.52 Hz), 7.66 (1 H, s), 11.12 (1 H, s), 12.72 (1 H, s)

### EXAMPLE 14

### 5-(2-chloro-6-fluorophenyl)-N-(2-methylimidazo[1,2-a]pyridin-3-yl)-1H-pyrazole-3-carboxamide

Obtained (7% yield) as a pale yellow solid from 5-(2-chloro-6-fluorophenyl)-1H-pyrazole-3-carboxylic acid (Preparation 3) and 2-methylimidazo[1,2-a]pyridin-3-amine (Preparation XX) following the experimental procedure as described in Example 1.
LRMS (m/z): 370 (M+1)⁺.
¹H NMR (300 MHz, DMSO-*d*₆) d ppm 2.28 (3 H, s), 6.88 (1 H, t, J=6.46 Hz), 7.03 (1 H, s), 7.16 - 7.28 (1 H, m), 7.34 - 7.69 (4 H, m), 7.98 (1 H, d, J=5.87 Hz), 10.28 (1 H, br. s), 13.91 (1 H, br. s)

### EXAMPLE 15

### 5-cyclohexyl-N-(1-methyl-3-(trifluoromethyl)-1H-pyrazol-5-yl)-1 H-pyrazole-3-carboxamide

Compound of Preparation 17 (100 mg, 0.5 mmol) and 1-methyl-3-(trifluoromethyl)-1H-pyrazol-5-amine (90 mg, 0.55 mmol) were dissolved in 2 mL DCM. 2-Benzotriazol-1-il-N,N,N',N'-tetramethyluronio hexafluorophosphate (226 mg, 0.6 mmol) and triethylamine (206 µL, 1.49 mmol) were added and the mixture was stirred at room temperature for 18 hours. Dichloromethane was added and the organic phase was washed with saturated aqueous NaHCO₃ solution, water and brine, dried over Na₂SO₄ and evaporated under reduced pressure and the residue was purified using the Isolera purification (ethyl acetate - hexane gradient, 0:100 rising to 100:0) to give 25 mg (0.07 mmol, 14%) of the title compound as an oil.
LRMS (m/z): 342 (M+1)⁺.
¹H NMR (300 MHz, DMSO-*d*₆) d ppm 0.97 - 1.53 (6 H ,m), 1.61 - 1.83 (3 H ,m), 1.85 - 2.07 (2H, m), 3.76 (3H, s), 6.54 (1 H, s), 6.63 (1 H, s), 10.28 (1 H , br. s), 13.22 (1 H, br. s)

### EXAMPLE 16

### 5-(2-chloro-6-fluorophenyl)-N-(2-methoxy-5-(methylcarbamoyl)phenyl)-1 H-pyrazole-3-carboxamide

To a solution of compound of Preparation 18 (155 mg, 0.86 mmol) and triethylamine (179 µL, 1.29 mmol) ) in 5 mL anhydrous dichloromethane, compound of Preparation 19 (267 mg, 1.03 mmol) is added and the mixture is stirred at room temperature for 48h. The reaction mixture is evaporated under reduced pressure and the residue was purified using the Isolera purification (dichloromethane:methanol (9:1) : dichloromethane gradient, 0:100 rising to 100:0) to give 181 mg (0.45 mmol, 52%) of the title compound.
LRMS (m/z): 403 (M+1)⁺.
¹H NMR (300 MHz, DMSO-*d*₆) d ppm 2.78 (3 H, d, J=4.70 Hz,), 3.97 (3H, s), 7.04 (1 H, br. s.), 7.18 (1 H ,d, J=8.80 Hz), 7.42-7.48 (1 H ,m), 7.53-7.64 (3H, m), 8.33-8.34 (1 H , m), 8.75 (1 H, br. s), 9.44 (1 H, br. s.), 13.88 (1 H, br. s)

### EXAMPLE 17

### 5-(2-chloro-6-fluorophenyl)-N-(5-(dimethylcarbamoyl)-2-methoxyphenyl)-1 H-pyrazole-3-carboxamide

Obtained (28% yield) as a solid from 3-amino-4-methoxy-N,N-dimethylbenzamide (Preparation 20) and 5-(2-chloro-6-fluorophenyl)-1H-pyrazole-3-carbonyl chloride (Preparation 19) following the experimental procedure as described in Example 17.
LRMS (m/z): 417 (M+1)⁺.
¹H NMR (300 MHz, DMSO- *d*₆) d ppm 2.99 (6H,s), 3.96 (3H, s), 7.05 (1 H, br. s.), 7.15-7.24 (2 H ,m), 7.42-7.47 (1 H, m), 7.49 - 7.68 (3H, m), 8.34 (1 H, br. s), 9.49 (1H,br.s)

### EXAMPLE 18

### 5-(2-chloro-6-fluorophenyl)-N-(2-methyl-5-sulfamoylphenyl)-1 H-pyrazole-3-carboxamide

To a solution of 3-amino-4-methylbenzenesulfonamide (80 mg, 0.43 mmol) and triethylamine (89 µL, 0.64 mmol) in 1 mL anhydrous dichloromethane, compound of Preparation 19 (133 mg, 0.52 mmol) is added and the mixture is stirred at 40°C overnight. The reaction mixture is evaporated under reduced pressure and the residue was purified using the Isolera purification (dichloromethane:methanol (9:1) : dichloromethane gradient, 0:100 rising to 100:0) to give 21 mg (0.05 mmol, 21 %) of the title compound.
LRMS (m/z): 409 (M+1)⁺.
¹H NMR (300 MHz, DMSO- *d*₆) d ppm 2.04 (3 H,s), 4.94 (2 H,br. s.), 6.48 (1 H ,s), 6.92 (2 H, q, J=8.02 Hz,), 7.12 (1 H, s), 7.29 (1 H, t, J=9.10 Hz,), 7.36 - 7.58 (2H, m)

### EXAMPLE 19

### 5-(2-chloro-6-fluorophenyl)-N-(5-(N,N-dimethylsulfamoyl)-2-methoxyphenyl)-1H-pyrazole-3-carboxamide

To a solution of 3-amino-4-methoxy-N,N-dimethylbenzenesulfonamide (80 mg, 0.35 mmol) and triethylamine (72 µL, 0.52 mmol) in 1 mL anhydrous dichloromethane, compound of Preparation 19 (108 mg, 0.42 mmol) is added and the mixture is stirred at 40°C overnight. The reaction mixture is evaporated under reduced pressure and the residue was purified using the Isolera purification (ethyl acetate - hexane gradient, 0:100 rising to 100:0) to give 110 mg (0.24 mmol, 70%) of the title compound.
LRMS (m/z): 453 (M+1)⁺.
¹H NMR (300 MHz, DMSO- *d*₆) d ppm 2.62 (6H, s), 4.04 (3H, s), 7.05 (1H, br. s), 7.36 (1 H, d, J=8.80 Hz), 7.37 - 7.70 (4H, m), 8.73 (1 H, br. s), 9.53 (1 H, br. s)

### EXAMPLE 20

### 5-(3-fluoropyridin-4-yl)-N-(1-methyl-3-(trifluoromethyl)-1 H-pyrazol-5-yl)-1 H-pyrazole-3-carboxamide

Compound of Preparation 22 (443 mg, 1.67 mmol) and 1-methyl-3-(trifluoromethyl)-1H-pyrazol-5-amine (706 mg, 4.28 mmol) were dissolved in 6 mL DMF. 2-Benzotriazol-1-il-N,N,N',N'-tetramethyluronio hexafluorophosphate (973 mg, 2.57 mmol) and triethylamine (889 µL, 6.41 mmol) were added and the mixture was stirred at room temperature for 18 hours. Dichloromethane was added and the organic phase was washed with saturated aqueous NaHCO₃ solution, water and brine, dried over Na₂SO₄ and evaporated under reduced pressure and the residue was purified using the Isolera purification (dichloromethane:methanol (9:1) : dichloromethane gradient,, 0:100 rising to 100:0) to give 35 mg (0.1 mmol, 6%) of the title compound.
LRMS (m/z): 355 (M+1)⁺.
1 H NMR (300 MHz, METHANOL-*d*₄) d ppm 3.77 (3H, s), 6.55 (1 H, s), 7.41 (1 H, s), 7.86 (1 H, s), 8.37-8.39 (1 H, m), 8.51-8.52 (1 H, m)

### EXAMPLE 21

### 2-(5-(2-chloro-6-fluorophenyl)-1 H-pyrazole-3-carboxamido)benzoic acid

Obtained (31% yield) as a solid from 2-aminobenzoic acid and 5-(2-chloro-6-fluorophenyl)-1H-pyrazole-3-carbonyl chloride (Preparation 19) following the experimental procedure as described in Example 17.
LRMS (m/z): 360 (M+1)⁺.
1 H NMR (300 MHz, DMSO-*d*₆) d ppm 7.02 (1H, s), 7.17-7.22 (1H, m), 7.37 - 7.50 (1 H, m), 7.50 - 7.74 (3H, m), 8.06 (1 H, d, J=6.46 Hz), 8.79 (1 H, d, J=7.63 Hz), 12.41 (1 H, br. s), 13.91 (1 H, br. s)

### EXAMPLE 22

### N-(5-carbamoyl-2-methoxyphenyl)-5-(2-chloro-6-fluorophenyl)-1 H-pyrazole-3-carboxamide

Obtained (3% yield) as a solid from 3-amino-4-methoxybenzamide and 5-(2-chloro-6-fluorophenyl)-1H-pyrazole-3-carbonyl chloride (Preparation 19) following the experimental procedure as described in Example 17.
LRMS (m/z): 389 (M+1)⁺.
¹H NMR (300 MHz, DMSO-*d*₆) d ppm 3.97 (3H, s), 6.97 - 7.33 (2H, m), 7.38 - 7.49 (1 H, m), 7.50 - 7.64 (2H, m), 7.70 (1 H, d, *J*=8.22 Hz), 7.89 (1 H, s), 8.71 (1 H, br. s), 9.48 (1 H,br. s)

### EXAMPLE 23

### 5-(2-chloro-6-fluorophenyl)-N-(1,3-dimethyl-1H-pyrazol-5-yl)-1H-pyrazole-3-carboxamide

Obtained (22% yield) as a solid from 1,3-dimethyl-1 H-pyrazol-5-amine and 5-(2-chloro-6-fluorophenyl)-1H-pyrazole-3-carbonyl chloride (Preparation 19) following the experimental procedure as described in Example 17.
LRMS (m/z): 334 (M+1)⁺.
¹H NMR (300 MHz, DMSO-d6) d ppm 2.13 (3H, s), 3.61 (3H, s), 6.00 (1H, br. s.), 6.99 (1 H, br. s), 7.18 - 7.85 (3H, m), 10.15 (1 H, br. s.), 13.85 (1 H, br. s)

### EXAMPLE 24

### 5-(2-chloro-6-fluorophenyl)-N-[1-ethyl-3-(trifluoromethyl)-1H-pyrazol-5-yl]-1H-pyrazole-3-carboxamide

Obtained from 1-ethyl-3-(trifluoromethyl)-1H-pyrazol-5-amine and 5-(2-chloro-6-fluorophenyl)-1H-pyrazole-3-carbonyl chloride (Preparation 19) following the experimental procedure as described in Example 17. The residue was purified using Isolera purification system (ethyl acetate - hexane gradient, 0:100 rising to 100:0) to give the title compound (42% yield) as a solid.
LRMS (m/z): 402 (M+1)⁺.
1 H NMR (300 MHz, DMSO-*d*₆) d ppm 1.37 (2 H, t, J=13.4 Hz), 4.13 (3 H, q, J=13.5 Hz), 6.71 (1 H, s), 7.03 (1 H, s), 7.31 - 7.68 (3 H, m), 10.50 (1 H, br. s)

### EXAMPLE 25

### 5-(2-chloro-6-fluorophenyl)-N-(2-methyl-2H-indazol-3-yl)-1H-pyrazole-3-carboxamide

Obtained (35% yield) as a solid from 2-methyl-2H-indazol-3-amine (Preparation 24) and 5-(2-chloro-6-fluorophenyl)-1H-pyrazole-3-carbonyl chloride (Preparation 19) following the experimental procedure as described in Example 17.
LRMS (m/z): 370 (M+1)⁺.
¹H NMR (300 MHz, DMSO-*d*₆) δ ppm 4.01 (3 H, s), 6.92 - 7.12 (2 H, m) 7.26 (1 H, t) 7.33 - 7.71 (5 H, m) 10.70 (1 H, br. s)

### EXAMPLE 26

### 5-(2-chloro-6-fluorophenyl)-N-(1-methyl-3-pyridin-4-yl-1H-pyrazol-5-yl)-1 H-pyrazole-3-carboxamide

Obtained (1% yield) as a solid from 5-(2-chloro-6-fluorophenyl)-1 H-pyrazole-3-carboxylic acid (Preparation 3) and 1-methyl-3-pyridin-4-yl-1 H-pyrazol-5-amine (Preparation 25) following the experimental procedure as described in Example 1.
LRMS (m/z): 397 (M+1)⁺.
¹H NMR (300 MHz, METHANOL-*d*₄) δ ppm 3.83 (3 H, s) 7.09 (1 H, br. s.) 7.22 - 7.34 (1 H, m) 7.41 - 7.55 (2 H, m) 7.64 (2 H, d, J=4.11 Hz) 7.91 - 8.07 (2 H, m), 8.46 (2 H, d, J=6.46 Hz)

### EXAMPLE 27

### 5-(2-chloro-6-fluorophenyl)-N-(1,4-dimethyl-3-phenyl-1H-pyrazol-5-yl)-1H-pyrazole-3-carboxamide

Obtained (35% yield) as a solid from 1,4-dimethyl-3-phenyl-1 H-pyrazol-5-amine and 5-(2-chloro-6-fluorophenyl)-1H-pyrazole-3-carbonyl chloride (Preparation 19) following the experimental procedure as described in Example 17.
LRMS (m/z): 410 (M+1)⁺.
¹H NMR (300 MHz, DMSO- *d*₆) δ ppm 2.06 (3 H, s), 3.70 (3 H, s), 7.07 (1 H, br. S.), 7.35 (1 H, d, J=7.04 Hz) 7.40 - 7.50 (3 H, m) 7.56 (3 H, t) 7.67 (2 H, d, J=7.04 Hz)

### EXAMPLE 28

### N-{[5-(2-chloro-6-fluorophenyl)-1H-pyrazol-3-yl]methyl}-1-methyl-3-(trifluoromethyl)-1H-pyrazol-5-amine

Compound of Example 1 (29 mg, 0.07 mmol) was dissolved in 2.0 mL tetrahydrofurane anhydrous. A 1.0 M Borane tetrahydrofuran complex solution (750 µL, 0.75 mmol) was added and the mixture was heated at 50°C in a sealed tube for 5 horas. 1 mL of a HCl 1.0N was added and the mixture was stirred for 1h at room temperature. Diethyl ether was added and the organic phase was washed with water two times, saturated aqueous NaHCO₃ solution and brine, dried over MgSO₄ and evaporated under reduced pressure and the residue was purified using the Isolera purification (dichlorometane-methanol/NH₃ gradient) to give 12 mg (0.03 mmol, 44%) of the title compound.
LRMS (m/z): 374 (M+1)⁺.
¹H NMR (300 MHz, DMSO-*d*₆) d ppm 3.63 (3 H, s), 4.28 (2 H, s), 6.42 (2 H, br. s), 7.23 - 7.57 (3 H, m)

### EXAMPLE 29

### 5-(2-chloro-6-fluorophenyl)-N-(2-methyl-4,5,6,7-tetrahydro-2H-indazol-3-yl)-1H-pyrazole-3-carboxamide

Obtained (23% yield) as a white solid from 5-(2-chloro-6-fluorophenyl)-1 H-pyrazole-3-carboxylic acid (Preparation 3) and 2-methyl-4,5,6,7-tetrahydro-2H-indazol-3-amine following the experimental procedure as described in Example 1.
LRMS (m/z): 374 (M+1)⁺.
¹H NMR (300 MHz, CHLOROFORM-*d*) d ppm 1.78 (4 H, m), 2.39 - 2.53 (2 H, m), 2.60 - 2.72 (2 H, m), 3.76 (3 H, s), 7.18 (1 H, m), 7.32 - 7.44 (2 H, m), 8.24 (1 H, s)

### BIOLOGICAL TESTS

### Fluorescent Calcium Flux Assay

### Cells:

Jurkat E6-1 cells were obtained from ATCC (number TIB-152™) and maintained as ATCC recommend; in medium RPMI-1640 with 10% FBS at 37°C and 5% CO₂.

### Assay:

Before the assay, Jurkat cells were grown at a density of 2 millions cells/ml in cell culture media (RPMI with 10% FBS) in a T-175 flask at 37°C and 5% CO2.

The day of the assay, cells were harvested by centrifugation (5 minutes at 1500 rpm) and the pellet were suspended in Ca²⁺ free assay buffer (HBSS w/o Ca²⁺ and Mg ²⁺, 20mM Hepes and 1 mM MgCl2, pH 7.4) at a density of 5 million cells/ml.

Calcium depletion was initiated by the addition to the cell suspension of Thapsigarguin at a final assay concentration of 10µM. 20µl of the cellular suspension containing Thapsigargin (100.000 cells/well) were plated into 384 well plates (corning 3711) and incubated at RT for 15 minutes.

Test compounds were diluted in DMSO 100% to get a 10⁻²M stock solution and serial dilutions 1/3 were performed in the same solvent. Then, 20µl of test compounds, diluted 75 times in Ca²⁺ free assay buffer, were added to the cells and incubated at RT for 1h.

Once this incubation was completed, 5 µl of dye solution (FLIPR Calcium 5 Express Kit, Molecular Devices), prepared as Molecular Devices recommend, were added into the assay plates followed by an extra incubation period of 1 hour at room temperature. After the second incubation period, assay plates were placed into the FLIPR Tetra from Molecular Devices. Fluorescence was measured at a wavelength of 525nm after the dye excitation at 485nm. The initial baseline was measured during 20 seconds, and then 8µl of a 13mM Calcium solution in assay buffer were added (2mM final calcium concentration) except for basal wells, where assay buffer without calcium was added. At this time, changes in the cellular fluorescent were measured for an extra 80 seconds.

Peak and base line were taken for the calculation of the ratio (Peak/Base Line). Total, basal and compound well ratio values were used to calculate %Inhibition.

### In vitro Assay of Cytokine Release from T Cells

Jurkat E6-1 cells were obtained from American Type Culture Collection (ATCC) and maintained in complete medium with 10% fetal bovine serum (FBS) at 37°C/5%CO₂. Cells were plated in 100 µL of Dubelcco's Modified Eagle Medium (DMEM) with 1% FBS in a 96 well plate at a density of 1x10⁶ cells/well and 50 µL of test compound was added. Cells were then stimulated by the addition of 50 µL of phytohemagglutinin (PHA) (final concentration 20 µg/ml) and incubated at 37°C/5%CO₂ for 20 hours. On the following day, the supernatants were collected and assayed for IL-2 levels by ELISA according to the manufacturer's protocols. The IC₅₀ value was calculated as the concentration at which 50% of secreted IL-2 in vehicle is inhibited.

| **Example** | **Ca²⁺ flux IC₅₀(nM)** | **Jurkat IL2 IC₅₀ (nM)** |
|---|---|---|
| Ex.1 | 332 | 130 |
| Ex.5 | 135 | 62 |
| Ex.6 | 559 | 410 |

As it can be seen form the table, compounds of the present invention are potent inhibitors of Ca²⁺ influx in Jurkat cells. Preferred compounds of the invention possess an IC₅₀ value for the inhibition of Ca²⁺ influx of less than 10 µM, preferably less than 1 µM, being most preferably less than 500 nM.

Compounds described herein have shown activity inhibiting IL2 secretion with an IC₅₀ value of less than 1 µM, being most preferably less than 500 nM.

### COMBINATIONS

The compounds of formula (I) can also be used in combination with other drugs known to be effective in the treatment of the diseases or the disorders indicated above. For example the compounds of the present invention can be combined with (a) anticholinergics, such as aclidinium bromide or tiotropium, (b) corticosteroids, or gluococorticoids such as prednisone or methylprednisolone, (c) antihistamines, such as ebastine, ranitidine, ABT-239 or JNJ 7777120; (d) beta-2 agonists, such as salmeterol or formoterol, (e) chemokine receptor antagonists, such as maraviroc or enfuvirtide, (f) CRth2 antagonists, (g) leukotriene receptor antagonists, (h) JAK inhibitors such as tofacitinib or INCB018424, (i) Syk inhibitors such as R-343, (j) phosphosdiesterase IV inhibitors such as roflumilast or revamilast, (k) dual beta-2 adrenoceptor agonist/M3 receptor antagonist (MABA compounds) such as GSK-961081, (I) p38 Inhibitors such as ARRY-797, (m) PKC inhibitors such as NVP-AEB071, (n) 5-lipoxygenase activating protein inhibitors, such as veliflapon, (o) 5-lipoxygenase inhibitors, (p) CYSLTR1 antagonists, such as montelukast, pranlukast or zafirlukast; (q) CYSLTR2 antagonists, such as pranlukast, zafirlukast or tipilukast; (r) BLT1 antagonists, (s) BLT2 antagonists, (t) thromboxane A2 antagonists such as ramatroban, (u) DP1 receptor antagonists, such as laropiprant, (v) DP1 receptor agonists, such as BW-245C, (w) IP receptor agonists, such as RO-1138452, (x) Anti-IgE, such as omalizumab, (y) IL5 antibody, such as mepolizumab, (z) leukotriene formation inhibitors, (aa) bronchodilators, such as pirbuterol, epinephrine, ephedrine or salbutamol; (bb) decongestants, such as ephedrine, levo-methamphetamine, naphazoline, oxymetazoline, phenylephrine, phenylpropanolamine, propylhexedrine, pseudoephedrine, synephrine or tetrahydrozoline; (cc) mucolytics such as acetylcysteine, ambroxol, bromhexine, carbocisteine, domiodol, eprazinone, erdosteine, letosteine, neltenexine, sobrerol, stepronin or tiopronin; (dd) antitussives, such as dextromethorphan, (ee) analgesics such as aspirin, paracetamol, rofecoxid, celecoxib, morphine, codeine, oxycodone, hydrocodone, dihydromorphine or flupirtine; and (ff) expectorants such antimony pentasulfide, guaiacolsulfonate, guaifenesin, potassium iodide or tyloxapol.

Accordingly, another embodiment of the invention is a combination product comprising (i) at least a compound as defined previously, and (ii) one or more active ingredients as described above, for simultaneous, separate or sequential use in the treatment of the human or animal body.

A preferred embodiment of the invention is a combination product as defined before for the treatment or prevention of pathological conditions, diseases and disorders known to be susceptible of amelioration by inhibition of CRAC channel activity, in particular wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease; as well as a method for treating a subject afflicted with a pathological condition or disease susceptible to amelioration by inhibition of CRAC channel activity in particular wherein the pathological condition or disease as described above; which comprises administering to said subject an effective amount of a combination product as defined before.

As indicated above, the compounds or pharmaceutically acceptable salts, N-oxides, or or isotopically-labeled derivates thereof, according to the invention may also be used in combination with another therapeutically active agent as defined above.

The amount of each active which is required to achieve a therapeutic effect will, of course, vary with the particular active, the route of administration, the subject under treatment, and the particular disease or disorder being treated.

The active ingredients may be administered from 1 to 6 times a day, sufficient to exhibit the desired activity. Preferably, the active ingredients are administered once or twice a day, most preferably once a day.

Examples of suitable (β2-agonists that can be combined with the compounds of formula (I) are terbutaline sulphate, eformoterol fumarate, formoterol fumarate, formoterol hydrochloride; bambuterol, ibuterol, isoprenaline hydrochloride, dopexamine, metaprotenerol, tulobuterol hydrochloride, procaterol hydrochloride, sibenadet hydrochloride, mabuterol hydrochloride, albuterol sulphate, salbutamol sulphate, salmefamol, salmeterol, salmeterol xinafoate, carmoterol, carmoterol hydrochloride, (R)-albuterol hydrochloride, levalbuterol hydrochloride; levosalbutamol hydrochloride; levalbuterol sulphate; levosalbutamol sulphate; (-)-salbutamol hydrochloride, formoterol, (R,R)-formoterol tartrate; arformoterol tartrate, sulfonterol, bedoradrine sulphate, indacaterol, indacaterol maleate, indacaterol acetate, indacaterol xinafoate, abediterol (LAS100977), abediterol napadislate, abediterol mesylate, trantinterol hydrochloride, milveterol hydrochloride, olodaterol, fenoterol hydrobromide, rimoterol hydrobromide, riproterol hydrochloride, vilanterol, broxaterol, pirbuterol hydrochloride, bitolterol mesylate, clenbuterol hydrochloride, AZD-3199, GSK-159802; GSK-597901, GSK-678007, GSK-961081; 4-[2-[3-(1 H-Benzimidazol-1-yl)-1,1-dimethylpropylamino]-1-hydroxyethyl]-2-(4-methoxybenzylamino)phenol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-domethoxyphenyl)-2-methyl-2-propylamino]ethanol, 1-[2H-5-hydroxy-3-oxo-4H-1,4-benzoxazin-8-yl]-2-[3-(4-n-butyloxyhenyl)-2-methyl-2-propylamino]ethanol, KUL-1248, KUL-7211; HOKU-81, SM-11044, RP-58802B; AR-C68164AA, AR-C68475AA; AR-C89855AA; AR-C69457AA; and compounds described in PCT patent applications Nos. WO 2007/124898, WO 2006/122788A1, WO 2008/046598, WO 2008095720, WO 2009/068177 and WO 2010/072354.

Examples of suitable corticosteroids and glucocorticoids that can be combined with the compounds of formula (I) are prednisolone, methylprednisolone, dexamethasone, dexamethasone acetate, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone, mometasone furoate, rimexolone, prednisolone farnesylate, ciclesonide, butixocort propionate, RS-85095, CGP-13774, GW-250495, deltacortisone, NO-Prednisolone, NO-Budesonide, etiprednol dicloacetate, QAE-397, 7beta-OH-EPIA, RPR-106541, deprodone propionate, fluticasone, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11 beta-hydroxy-17alpha-[2-(methylsulfanyl)acetoxy]-4-pregnene-3,20-dione, desisobutyrylciclesonide, hydrocortisone acetate, hydrocortisone sodium succinate, prednisolone sodium phosphate and hydrocortisone probutate, prednisolone sodium metasulfobenzoate and clobetasol propionate.

Examples of suitable M3 antagonists (anticholinergics) that can be combined with the compounds of formula (I) are tiotropium salts, oxitropium salts, flutropium salts, ipratropium salts, glycopyrronium salts, trospium salts, zamifenacin, revatropate, espatropate, darotropium bromide, Cl-923, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1 (R)-cyclopentyl]-2-hydroxy-2-phenylacetamide (J-1 04135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1 (R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1-methyl-1-[2-oxo-2-(3-thienyl)ethyl]pyrrolidinium iodide, N-[1-(3-Hydroxybenzyl)-1-methylpiperidinium-3(S)-yl]-N-[N-[4-(isopropoxycarbonyl)phenyl]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-fluorophenyl)-2-oxoimidazolidin-1-yl]-1-methyl-1-(2-phenylethyl)pyrrolidinium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Examples of suitable are anti-histamines that can be combined with the compounds of formula (I) are methapyrilene, mequitazine, azelastine hydrochloride, acrivastine, emedastine difumarate, emedastine fumarate, loratadine, cyproheptadine hydrochloride, diphenhydramine hydrochloride, doxepin hydrochloride, promethazine hydrochloride, levocabastine hydrochloride, desloratadine, cinnarizine, setastine hydrochloride, mizolastine, ebastine, cetirizine hydrochloride, epinastine hydrochloride, olopatadine hydrochloride, bepotastine besilate, triprolidine hydrochloride, rupatadine fumarate, fexofenadine hydrochloride, levocetirizine dihydrochloride, ketotifen, azatadine maleate, dimethindene maleate, clemastine fumarate, alcaftadine, bilastine, vapitadine hydrochloride, AZD-1744, GSK-1004723D, GSK-835726 or SUN-1334H.

Examples of suitable PDE4 inhibitors that can be combined with the compounds of formula (I) are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, revamilast, ronomilast, (R)-(+)-4-[2-(3-Cyclopentyloxy-4-methoxyphenyl)-2-phenylethyl]pyridine (CDP-840), N-(3,5-Dichloro-4-pyridinyl)-2-[1-(4-fluorobenzyl)-5-hydroxy-1 H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxyethyl)pyridin-2(1H)-one (T-440), (-)-trans-2-[3'-[3-(N-Cyclopropylcarbamoyl)-4-oxo-1,4-dihydro-1,8-naphthyridin-1-yl]-3-fluorobiphenyl-4-yl]cyclopropanecarboxylic acid, MK-0873, CDC-801, GSK-356278, TA-7906, CP-80633, RPL-554, NIK-616, GPD-1116, D4396, UK-500001, BLX-914, 2-carbomethoxy-4-cyano-4-(3-cyclopropylmethoxy-4-difluroromethoxyphenyl) cyclohexan1-one, cis [4-cyano-4-(3-cyclopropylmethoxy-4-difluoromethoxy-phenyl)cyclohexan-1-ol, 5(S)-[3-(Cyclopentyloxy)-4-methoxyphenyl]-3(S)-(3-methyl-benzyl)piperidin-2-one (IPL-455903), ONO-6126 (Eur Respir J 2003, 22(Suppl. 45): Abst 2557) and the compounds claimed in the PCT patent applications number WO 03/097613, WO 2004/058729, WO 2005/049581, WO 2005/123693, WO 2005/123692, and WO 2010/069504.

Examples of suitable leukotriene antagonist that can be combined with the compounds of formula (I) are CYSLTR1 antagonists, such as montelukast, pranlukast or zafirlukast; or CYSLTR2 antagonists, such as pranlukast, zafirlukast or tipilukast.

Examples of suitable CRTH₂ antagonist that can be combined with the compounds of formula (I) are ramatroban, AMG-009, OC-000459).

Examples of suitable Syk kinase inhibitors that can be combined with the compounds of formula (I) are fosfamatinib (from Rigel), R-348 (from Rigel), R-343 (from Rigel), R-112 (from Rigel), piceatannol, 2-(2-Aminoethylamino)-4-[3-(trifluoromethyl)phenylamino] pyrimidine-5-carboxamide, R-091 (from Rigel), 6-[5-Fluoro-2-(3,4,5-trimethoxyphenylamino)pyrimidin-4-ylamino]-2,2-dimethyl-3,4-dihydro-2H-pyrido[3,2-b][1,4]oxazin-3-one benzenesulfonate (R-406 from Rigel), 1-(2,4,6-Trihydroxyphenyl)-2-(4-methoxyphenyl)ethan-1-one, N-[4-[6-(Cyclobutylamino)-9H-purin-2-ylamino]phenyl]-N-methylacetamide (QAB-205 from Novartis), CI-1002 (from Pfizer), VRT-750018 (from Vertex), PRT-062607, 2-[7-(3,4-Dimethoxyphenyl)imidazo[1,2-c]pyrimidin-5-ylamino]pyridine-3-carboxamide dihydrochloride (BAY-61-3606 from Bayer) and AVE-0950 (from Sanofi-Aventis).

The compounds of formula (I) and the combinations of the invention may be used in the treatment of respiratory, skin and inflammatory diseases, wherein the use of a CRAC modulador channel inhibitor is expected to have a beneficial effect, for example an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease

The active compounds in the combination product may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be administered in the morning and the other (s) later in the day. Or in another scenario, one or two actives could be administered twice daily and the other (s) once daily, either at the same time as one of the twice-a-day dosing occurred, or separately. Preferably at least two, and more preferably all, of the actives would be administered together at the same time. Preferably, at least two, and more preferably all actives would be administered as an admixture.

### PHARMACEUTICAL COMPOSITIONS

Compounds of the invention intended for pharmaceutical use may be administered as crystalline or amorphous products, or mixtures thereof. They may be obtained, for example, as solid plugs, powders, or films by methods such as precipitation, crystallization, freeze drying, spray drying, or evaporative drying. Microwave or radio frequency drying may be used for this purpose.

Pharmaceutical compositions according to the present invention comprise the compounds of the invention in association with a pharmaceutically acceptable diluent or carrier.

As used herein, the term pharmaceutical composition refers to a mixture of one or more of the compounds described herein, or physiologically/pharmaceutically acceptable salts, or N-oxides, or isotopically-labeled derivates thereof, with other chemical components, such as physiologically/pharmaceutically acceptable carriers and excipients. The purpose of a pharmaceutical composition is to facilitate administration of a compound to an organism.

As used herein, a physiologically/pharmaceutically acceptable diluent or carrier refers to a carrier or diluent that does not cause significant irritation to an organism and does not abrogate the biological activity and properties of the administered compound.

A pharmaceutically acceptable excipient refers to an inert substance added to a pharmaceutical composition to further facilitate administration of a compound.

The invention further provides pharmaceutical compositions comprising the compounds of the invention in association with a pharmaceutically acceptable diluent or carrier together with one or more other therapeutic agents such as the previously described for use in the treatment of a pathological condition or disease susceptible to amelioration by inhibition of CRAC channel modulators.

The invention is also directed to pharmaceutical compositions of the invention for use in the treatment of a pathological disease or disorder susceptible to amelioration by inhibition of CRAC channel modulators, in particular wherein the pathological disease or disorder is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease.

The invention also provides a method of treatment of a pathological condition or disease susceptible to amelioration by inhibition of CRAC channel modulators, in particular wherein the pathological condition or disease as defined above, comprising administering a therapeutically effective amount of a pharmaceutical composition of the invention.

The present invention also provides pharmaceutical compositions which comprise, as an active ingredient, at least a compound of formula (I) or a pharmaceutically acceptable salt, solvate, N-oxide or deuterated derivative thereof in association with a pharmaceutically acceptable excipient such as a carrier or diluent. The active ingredient may comprise 0.001% to 99% by weight, preferably 0.01% to 90% by weight, of the composition depending upon the nature of the formulation and whether further dilution is to be made prior to application. Preferably the compositions are made up in a form suitable for oral, inhalation, topical, nasal, rectal, percutaneous or injectable administration.

Pharmaceutical compositions suitable for the delivery of compounds of the invention and methods for their preparation will be readily apparent to those skilled in the art. Such compositions and methods for their preparation can be found, for example, in Remington: The Science and Practice of Pharmacy, 21 st Edition, Lippincott Williams & Wilkins, Philadelphia, Pa., 2001.

The pharmaceutically acceptable excipients which are admixed with the active compound or salts of such compound, to form the compositions of this invention are well-known per se and the actual excipients used depend inter alia on the intended method of administering the compositions. Examples, without limitation, of excipients include calcium carbonate, calcium phosphate, various sugars and types of starch, cellulose derivatives, gelatin, vegetable oils and polyethylene glycols.

Additional suitable carriers for formulations of the compounds of the present invention can be found in Remington: The Science and Practice of Pharmacy, 21st Edition, Lippincott Williams & Wilkins, Philadelphia, Pa., 2001; or in Handbook of Pharmaceutical Excipients, 6th ed., published by Pharmaceutical Press and American Pharmacists Association, 2009.

### i) Oral Administration

The compounds of the invention may be administered orally (peroral administration; *per* os (latin)). Oral administration involve swallowing, so that the compound is absorbed from the gut and delivered to the liver via the portal circulation (hepatic first pass metabolism) and finally enters the gastrointestinal (GI) tract.

Compositions for oral administration may take the form of tablets, retard tablets, sublingual tablets, capsules, inhalation aerosols, inhalation solutions, dry powder inhalation, or liquid preparations, such as mixtures, solutions, elixirs, syrups or suspensions, all containing the compound of the invention; such preparations may be made by methods well-known in the art. The active ingredient may also be presented as a bolus, electuary or paste.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose. A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For tablet dosage forms, depending on dose, the drug may make up from 1 wt% to 80 wt% of the dosage form, more typically from 5 wt% to 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl- substituted hydroxypropyl cellulose, starch, pregelatinized starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinized starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate. Tablets may also optionally include surface active agents, such as sodium lauryl sulfate and polysorbate 80, and glidants such as silicon dioxide and talc. When present, surface active agents are typically in amounts of from 0.2 wt% to 5 wt% of the tablet, and glidants typically from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally are present in amounts from 0.25 wt% to 10 wt%, preferably from 0.5 wt% to 3 wt% of the tablet. Other conventional ingredients include anti-oxidants, colorants, flavoring agents, preservatives and taste-masking agents.

Exemplary tablets contain up to about 80 wt% drug, from about 10 wt% to about 90 wt% binder, from about 0 wt% to about 85 wt% diluent, from about 2 wt% to about 10 wt% disintegrant, and from about 0.25 wt% to about 10 wt% lubricant. Tablet blends may be compressed directly or by roller to form tablets. Tablet blends or portions of blends may alternatively be wet-, dry-, or melt-granulated, melt congealed, or extruded before tabletting. The final formulation may include one or more layers and may be coated or uncoated; or encapsulated.

The formulation of tablets is discussed in detail in "Pharmaceutical Dosage Forms: Tablets, Vol. 1 ", by H. Lieberman and L. Lachman, Marcel Dekker, N.Y., 1980.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Solid formulations for oral administration may be formulated to be immediate and/or modified release. Modified release formulations include delayed-, sustained-, pulsed-, controlled-, targeted and programmed release.

Liquid formulations include suspensions, solutions, syrups and elixirs. Such formulations may be used as fillers in soft or hard capsules and typically include a carrier, for example, water, ethanol, polyethylene glycol, propylene glycol, methylcellulose, or a suitable oil, and one or more emulsifying agents and/or suspending agents. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent. Liquid formulations may also be prepared by the reconstitution of a solid, for example, from a sachet.

### ii) Oral mucosal administration

The compounds of the invention can also be administered via the oral mucosal. Within the oral mucosal cavity, delivery of drugs is classified into three categories: (a) sublingual delivery, which is systemic delivery of drugs through the mucosal membranes lining the floor of the mouth, (b) buccal delivery, which is drug administration through the mucosal membranes lining the cheeks (buccal mucosa), and (c) local delivery, which is drug delivery into the oral cavity.

Pharmaceutical products to be administered via the oral mucosal can be designed using mucoadhesive, quick dissolve tablets and solid lozenge formulations, which are formulated with one or more mucoadhesive (bioadhesive) polymers (such as hydroxy propyl cellulose, polyvinyl pyrrolidone, sodium carboxymethyl cellulose, hydroxy propyl methyl cellulose, hydroxy ethyl cellulose, polyvinyl alcohol, polyisobutylene or polyisoprene); and oral mucosal permeation enhancers (such as butanol, butyric acid, propranolol, sodium lauryl sulphate and others)

### iii) Inhaled administration

The compounds of the invention can also be administered by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomizer (preferably an atomizer using electrohydrodynamics to produce a fine mist), or nebulizer, with or without the use of a suitable propellant, such as 1 ,1 ,1 ,2-tetrafluoroethane or 1 ,1 ,1 ,2,3,3,3-heptafluoropropane. For intranasal use, the powder may include a bioadhesive agent, for example, chitosan or cyclodextrin.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 0.001-5000 mg, more preferably 0.01-1000 mg of active ingredient or the equivalent amount of a pharmaceutically acceptable salt thereof. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (Ex. EP0069715) or disks (Ex. GB 2041763; EP 0424790; DE 4239402 and EP 0674533), rotatable cylinders (Ex. EP 0166294; GB 2165159 and WO 92/09322) and rotatable frustums (Ex. WO 92/00771), all having cavities which have to be filled with powder from the container. Other multi dose devices have measuring slides (Ex. US 5201308 and WO 97/00703) or measuring plungers with a local or circumferential recess to displace a certain volume of powder from the container to a delivery chamber or an air conduit (Ex. EP 0505321, WO 92/04068 and WO 92/04928), or measuring slides such as the Genuair® (formerly known as Novolizer SD2FL), which is described the following patent applications Nos: WO97/000703, WO03/000325 and WO2006/008027.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (Ex. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of socalled atomisers, via which solutions of pharmacologically-active substances can be sprayed under high pressure so that a mist of inhalable particles results. The advantage of these atomisers is that the use of propellant gases can be completely dispensed with. Such atomiser is the respimat® which is described, for example, in PCT Patent Applications Nos. WO 91/14468 and WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetra-fluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants (eg oleic acid or lecithin) and cosolvens (eg ethanol). Pressurised formulations will generally be retained in a canister (e.g. an aluminium canister) closed with a valve (e.g. a metering valve) and fitted into an actuator provided with a mouthpiece.

Medicaments for administration by inhalation desirably have a controlled particle size. The optimum particle size for inhalation into the bronchial system is usually 1-10 µm, preferably 2-5 µm. Particles having a size above 20 µm are generally too large when inhaled to reach the small airways. To achieve these particle sizes the particles of the active ingredient as produced may be size reduced by conventional means e.g. by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate.
Pressurized aerosol compositions will generally be filled into canisters fitted with a valve, especially a metering valve. Canisters may optionally be coated with a plastics material e. g. a fluorocarbon polymer as described in WO96/32150. Canisters will be fitted into an actuator adapted for buccal delivery.

### iv) Nasal mucosal administration

The compounds of the invention may also be administered via the nasal mucosal.

Typical compositions for nasal mucosa administration are typically applied by a metering, atomizing spray pump and are in the form of a solution or suspension in an inert vehicle such as water optionally in combination with conventional excipients such as buffers, anti-microbials, tonicity modifying agents and viscosity modifying agents.

### v) Parenteral Administration

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water.

The preparation of parenteral formulations under sterile conditions, for example, by lyophilization, may readily be accomplished using standard pharmaceutical techniques well known to those skilled in the art. The solubility of compounds of the invention used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

### vi) Topical Administration

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated; see, for example, J Pharm Sci, 88 (10), 955-958 by Finnin and Morgan (October 1999). Other means of topical administration include delivery by electroporation, iontophoresis, phonophoresis, sonophoresis and microneedle or needle-free injection.

Formulations for topical administration may be formulated to be immediate and/or modified release. Modified release formulations include delayed-, sustained-, pulsed-, controlled-, targeted and programmed release.

### vii) Rectal/intravaginal Administration

Compounds of the invention may be administered rectally or vaginally, for example, in the form of a suppository, pessary, or enema. Cocoa butter is a traditional suppository base, but various alternatives may be used as appropriate. Formulations for rectal/vaginal administration may be formulated to be immediate and/or modified release. Modified release formulations include delayed-, sustained-, pulsed-, controlled-, targeted and programmed release.

### viii) Ocular Administration

Compounds of the invention may also be administered directly to the eye or ear, typically in the form of drops of a micronized suspension or solution in isotonic, pH-adjusted, sterile saline. Other formulations suitable for ocular and aural administration include ointments, biodegradable {e.g. absorbable gel sponges, collagen) and nonbiodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

### ix) Other Technologies

Compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

The amount of the active compound administered will be dependent on the subject being treated, the severity of the disorder or condition, the rate of administration, the disposition of the compound and the discretion of the prescribing physician. However, an effective dosage is typically in the range of 0.01-3000 mg, more preferably 0.5-1000 mg of active ingredient or the equivalent amount of a pharmaceutically acceptable salt thereof per day. Daily dosage may be administered in one or more treatments, preferably from 1 to 4 treatments, per day.

The pharmaceutical formulations may conveniently be presented in unit dosage form and may be prepared by any of the methods well known in the art of pharmacy.

Preferably the composition is in unit dosage form, for example a tablet, capsule or metered aerosol dose, so that the patient may administer a single dose.

The active substance compositions according to the invention are preferably administered in the form of compositions for inhalation delivered with the help of inhalers, especially dry powder inhalers; however, any other form of nasal, topical, parenteral or oral application is possible. Here, the application of inhaled compositions embodies one of the preferred application form, especially in the therapy of obstructive lung diseases or for the treatment of asthma. Other preferred application form is dermal and transdermal administration, especially in the therapy of atopic dermatitis or psoriasis.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

### FORMULATION EXAMPLES

The following preparations forms are cited as formulation examples:

### Formulation Example 1 (Oral suspension)

| **Ingredient** | **Amount** |
|---|---|
| Active Compound | 3 mg |
| Citric acid | 0,5 g |
| Sodium chloride | 2,0 g |
| Methyl paraben | 0,1 g |
| Granulated sugar | 25 g |
| Sorbitol (70% solution) | 11 g |
| Veegum K | 1,0 g |
| Flavoring | 0,02 g |
| Dye | 0,5 mg |
| Distilled water | q.s. to 100 mL |

### Formulation Example 2 (Hard gelatine capsule for oral administration)

| **Ingredient** | **Amount** |
|---|---|
| Active Compound | 1 mg |
| Lactose | 150 mg |
| Magnesium stearate | 3 mg |

### Formulation Example 3 (Gelatin cartridge for inhalation)

| **Ingredient** | **Amount** |
|---|---|
| Active Compound (micronized) | 0,2 mg |
| Lactose | 25 mg |

### Formulation Example 4 (Formulation for inhalation with a DPI)

| **Ingredient** | **Amount** |
|---|---|
| Active Compound (micronized) | 15 mg |
| Lactose | 3000 mg |

### Formulation Example 5 (Formulation for a MDI)

| **Ingredient** | **Amount** |
|---|---|
| Active Compound (micronized) | 10 g |
| 1,1,1,2,3,3,3-heptafluoro-n-propane | q.s. to 200 ml |

Modifications, which do not affect, alter, change or modify the essential aspects of the compounds, combinations or pharmaceutical compositions described, are included within the scope of the present invention.

## Claims

1. A compound of formula (I), or pharmaceutically acceptable salts, N-oxides, or isotopically-labeled derivatives thereof. 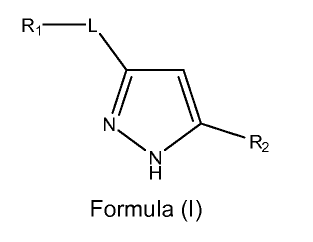 wherein:
L is selected from the group consisting of -CO-NH-, -NH-CO- and -NH-CH₂- group, wherein in the case of -NH-CH₂- group, the -CH₂- moiety is attached to the pyrazolyl ring while the -NH- moiety is attached to the R₁ substituent,
R₁ is selected from the group consisting of: 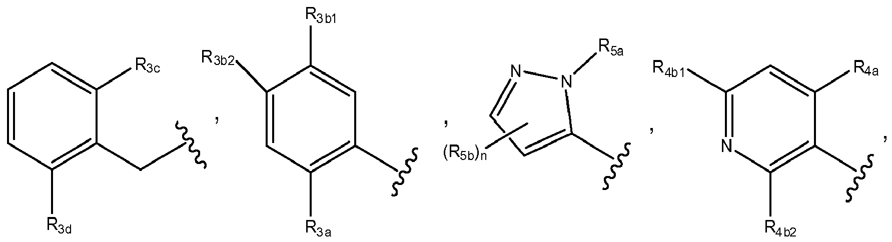 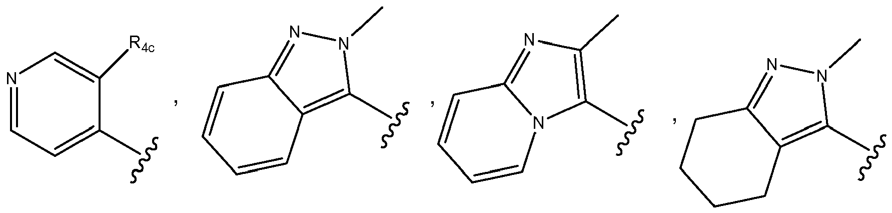 wherein
R₃ₐ is independently selected from the group consisting of a halogen atom, a-COOH group, a C₁-₄ alkyl group and a C₁-₄ alkoxy group,
R_{3b1} and R_{3b2} independently are selected from the group consisting of a hydrogen atom, a halogen atom, a C₁-₄ alkyl group, a C₁-₄ alkoxy group, a -CO-NR'R" group and a -SO₂-NR'R" group, wherein R' and R" are independently selected from the group consisting of a hydrogen atom, and a C₁-₄ alkyl group, R_{3c} and R_{3d} are independently selected from the group consisting of halogen atom, C₁-₄ alkyl group and C₁-₅ alkoxy group,
R₄ₐ is selected from the group consisting of a hydrogen atom, a halogen atom, a C₁-₄ alkyl group and a C₁-₄ alkoxy group,
R_{4b1} and R_{4b2} independently are selected from the group consisting of a hydrogen atom, a C₁-₄ alkyl group and a C₁-₄ alkoxy group,
R_{4c} is selected from the group consisting of a halogen atom, a C₁-₄ alkyl group and a C₁-₄ alkoxy group
R₅ₐ represents a C₁-₄ alkyl group or a C₃-₆ cycloalkyl group, wherein the alkyl and the cycloalkyl groups are optionally substituted with one or more fluorine atoms.
R_{5b} is independently selected from the group consisting of a C₁-₄ alkyl group, a C₃-₆ cycloalkyl group, a C₅-₈ aryl group and a 5-to 8-membered heteroaryl group containing at least one heteroatom selected from N, S and O, wherein the alkyl group is optionally substituted with one or more fluorine atom,
R₂ is selected from the group consisting of: 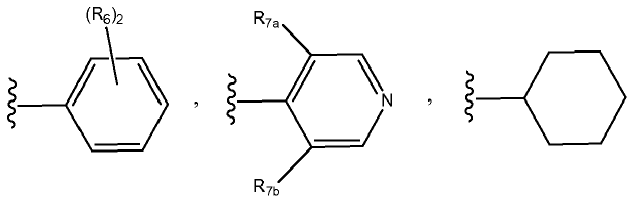 Wherein:
R₆ is selected from the group consisting of a halogen atom, a C₁-₄ alkyl group, and a C₁-₄ alkoxy group,
R₇ₐ and R_{7b} independently represent a hydrogen atom, a halogen atom or a C₁-₂ alkyl group, with the proviso that both R₇ₐ and R_{7b} cannot be a hydrogen atom,
n has a value of 1, 2 or 3,
wherein the compound of formula (I) is not 2-chloro-5-(N,N-diethylsulfamoyl)-N-(5-(2,6-difluorophenyl)-1 H-pyrazol-3-yl)benzamide, N-(5-(5-bromo-2-chlorophenyl)-1 H-pyrazol-3-yl)-2-fluorobenzamide nor N-(5-(2,6-difluorophenyl)-1 H-pyrazol-3-yl)-1,3-dimethyl-1 H-pyrazole-5-carboxamide.

2. A compound according to claim 1, wherein L is selected from the group consisting of -CO-NH-, -NH-CO-, preferably, -NH-CO-, wherein the -CO- moiety is attached to the pyrazolyl ring while the -NH- moiety is attached to the R₁ substituent.

3. A compound according to claim 1 or 2, wherein R₁ is selected from the group consisting of: 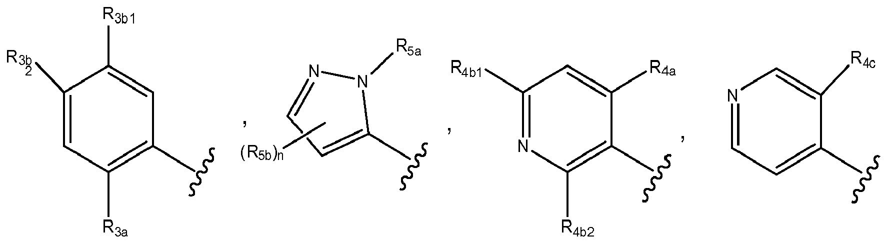 wherein R₃ₐ, R_{3b1}, R_{3b2}, R₄ₐ, R_{4b1}, R_{3b2}, R_{4c}, R₅ₐ, R_{5b} and n are as defined in claim 1.

4. A compound according to claim 3, wherein R₁ is selected from the group consisting of: 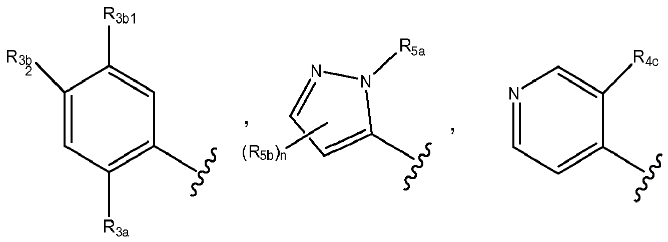 wherein R₃a, R_{3b1}, R_{3b2}, R_{4c}, R₅ₐ, R_{5b} and n are as defined in claim 1.

5. A compound according to claim 4, wherein R₁ is selected from the group consisting of: 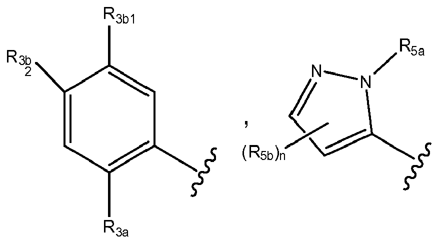 wherein R₃ₐ, R_{3b1}, R_{3b2}, R₅ₐ, R_{5b} and n are as defined in claim 1.

6. A compound according to any preceding claims, wherein:
(a) R₃ₐ represents a halogen atom or a C₁-₄ alkoxy group, preferably a C₁₋₄ alkoxy group, more preferably a metoxy group, and/or
(b) R_{3b1} and R_{3b2} independently are selected from the group consisting of a hydrogen atom, a halogen atom, a C₁-₄ alkoxy group, a -CO-NR'R" group and a -SO₂-NR'R" group, wherein R' and R" are independently selected from the group consisting of a hydrogen atom, and a methyl group, preferably R_{3b1} is selected from the group consisting of a methoxy group, a -CO-NR'R" group and a -SO₂-NR'R" group, wherein both R' and R" represents a methyl group while R_{3b2} represents a hydrogen atom, more preferably, R_{3b1} represents a methoxy group while R_{3b2} represents a hydrogen atom,.

7. A compound according to claim 1 or 2, wherein both R_{3c} and R_{3d} represent a halogen atom or a methoxy group, preferably both R_{3c} and R_{3d} represent a chlorine atom.

8. A compound according to any one of claims 1-3, wherein
(a) R₄ₐ is selected from the group consisting of a hydrogen atom and a C₁-₄ alkyl group, preferably a hydrogen atom or a methyl group, and/or
(b) R_{4b1} and R_{4b2} independently are selected from the group consisting of a hydrogen atom, a methyl group and a methoxy group, preferably R_{4b1} represents a hydrogen atom or a methyl group while R_{4b2} represents a methoxy group.

9. A compound according to any one of claims 1-5, wherein
(a) R₅ₐ represents an non-substituted C₁-₃ alkyl group, preferably a methyl or an ethyl group, and/or
(b) R_{5b} is selected from the group consisting of a C₁-₄ alkyl group and a C₅-₈ aryl group, wherein the alkyl group is optionally substituted with one or more fluorine atom, preferably, R_{5b} represents a -CF₃ group or a phenyl group.

10. A compound according to claim 9, wherein n has a value of 1 or 2, preferably 1.

11. A compound according to any preceding claims, wherein R₂ is selected from the group consisting of: 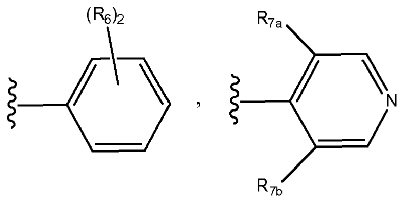 wherein R₆, R₇ₐ and R_{7b} are as defined in claim 1, preferably, both R₇ₐ represents a halogen atom and R_{7b} a hydrogen atom, more preferably, R₇ₐ represents a fluorine atom.

12. A compound according to claim 11, wherein R₂ represents a phenyl group substituted with two substituents selected from halogen atom and methoxy group, preferably halogen atoms, more preferably chlorine and fluorine atoms.

13. A compound according to claim 1, wherein:
R₃ₐ is independently selected from the group consisting of a -COOH group, a methyl group and a methoxy group,
R_{3b1} is selected from the group consisting of a hydrogen atom, methoxy group,
a -CO-NR'R" group and a -SO₂-NR'R" group, wherein R' and R" are independently selected from the group consisting of a hydrogen atom and a methyl group,
R_{3b2} represents a hydrogen atom,
Both R_{3c} and R_{3d} represent a chlorine atom,
R₄ₐ is selected from the group consisting of a hydrogen atom and a methyl group,
R_{4b1} is selected from the group consisting of a hydrogen atom and a methyl group,
R_{4b2} represents a metyoxy group,
R_{4c} represents a fluorine atom,
R₅ₐ represents a methyl or an ethyl group,
R_{5b} is selected from the group consisting of a methyl group, -CF₃ group, a phenyl group and a pyridyl group,
R₆ is selected from the group consisting of a fluorine atom, a chlorine atom and
a methoxy group,
R₇ₐ represents a hydrogen atom and R_{7b} represents a fluorine atom,
n has a value of 1 or 2,

14. A compound according to claim 1, wherein
L represents -NH-CO- group, wherein the -CO- moiety is attached to the pyrazolyl ring while the -NH- moiety is attached to the R₁ substituent
R₁ represents 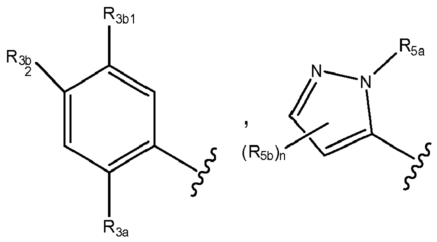 wherein
R₃ₐ represents a methoxy group,
R_{3b}, represents a methoxy group while R_{3b2} a hydrogen atom,
R₅ₐ represents a methyl or an ethyl group,
R_{5b} represents a -CF₃ group or a phenyl group.
R₂ represents a phenyl group substituted with two substituents selected from chlorine and fluorine atoms.

15. A compound according to claim 1, which is one of:
5-(2-chloro-6-fluorophenyl)-*N*-[1-methyl-3-(trifluoromethyl)-1*H*-pyrazol-5-yl]-1*H-*pyrazole-3-carboxamide
*N*-[5-(2-chloro-6-fluorophenyl)-1*H*-pyrazol-3-yl]-1-methyl-3-(trifluoromethyl)-1*H-*pyrazole-5-carboxamide
5-(2-chloro-6-fluorophenyl)-*N*-(6-methoxy-4-methylpyridin-3-yl)-1*H*-pyrazole-3-carboxamide
5-(2-chloro-6-fluorophenyl)-*N*-(2,6-dichlorobenzyl)-1*H*-pyrazole-3-carboxamide
5-(2-chloro-6-fluorophenyl)-*N*-(2,5-dimethoxyphenyl)-1*H*-pyrazole-3-carboxamide
5-(2-chloro-6-fluorophenyl)-*N*-(1-methyl-3-phenyl-1*H*-pyrazol-5-yl)-1*H*-pyrazole-3-carboxamide
5-(2-chloro-6-fluorophenyl)-*N*-(6-methoxy-2-methylpyridin-3-yl)-1*H*-pyrazole-3-carboxamide
5-(2,5-dimethoxyphenyl)-*N*-(3-fluoropyridin-4-yl)-1*H*-pyrazole-3-carboxamide
5-(2,5-dimethoxyphenyl)-*N*-[1-methyl-3-(trifluoromethyl)-1*H*-pyrazol-5-yl]-1*H-*pyrazole-3-carboxamide
*N*-[5-(2,5-dimethoxyphenyl)-1*H*-pyrazol-3-yl]-3-fluoroisonicotinamide
5-(2-chloro-6-fluorophenyl)-*N*-(3-fluoropyridin-4-yl)-1*H*-pyrazole-3-carboxamide
*N*-[5-(2-chloro-6-fluorophenyl)-1*H*-pyrazol-3-yl]-2,5-dimethoxybenzamide
*N*-[5-(2,5-dimethoxyphenyl)-1*H*-pyrazol-3-yl]-1-methyl-3-(trifluoromethyl)-1*H-*pyrazole-5-carboxamide
5-(2-chloro-6-fluorophenyl)-*N*-(2-methylimidazo[1,2-a]pyridin-3-yl)-1*H*-pyrazole-3-carboxamide
5-cyclohexyl-N-(1-methyl-3-(trifluoromethyl)-1H-pyrazol-5-yl)-1H-pyrazole-3-carboxamide
5-(2-chloro-6-fluorophenyl)-N-(2-methoxy-5-(methylcarbamoyl)phenyl)-1H-pyrazole-3-carboxamide
5-(2-chloro-6-fluorophenyl)-N-(5-(dimethylcarbamoyl)-2-methoxyphenyl)-1 H-pyrazole-3-carboxamide
5-(2-chloro-6-fluorophenyl)-N-(2-methyl-5-sulfamoylphenyl)-1H-pyrazole-3-carboxamide
5-(2-chloro-6-fluorophenyl)-N-(5-(N,N-dimethylsulfamoyl)-2-methoxyphenyl)-1H-pyrazole-3-carboxamide
5-(3-fluoropyridin-4-yl)-N-(1-methyl-3-(trifluoromethyl)-1H-pyrazol-5-yl)-1H-pyrazole-3-carboxamide
2-(5-(2-chloro-6-fluorophenyl)-1H-pyrazole-3-carboxamido)benzoic acid
N-(5-carbamoyl-2-methoxyphenyl)-5-(2-chloro-6-fluorophenyl)-1H-pyrazole-3-carboxamide
5-(2-chloro-6-fluorophenyl)-N-(1,3-dimethyl-1H-pyrazol-5-yl)-1H-pyrazole-3-carboxamide
5-(2-chloro-6-fluorophenyl)-*N*-[1-ethyl-3-(trifluoromethyl)-1*H*-pyrazol-5-yl]-1*H-*pyrazole-3-carboxamide
5-(2-chloro-6-fluorophenyl)-*N*-(2-methyl-2*H*-indazol-3-yl)-1*H-*pyrazole-3-carboxamide
5-(2-chloro-6-fluorophenyl)-*N*-(1-methyl-3-pyridin-4-yl-1*H*-pyrazol-5-yl)-1*H-*pyrazole-3-carboxamide
5-(2-chloro-6-fluorophenyl)-*N*-(1,4-dimethyl-3-phenyl-1*H*-pyrazol-5-yl)-1*H-*pyrazole-3-carboxamide
*N*-{[5-(2-chloro-6-fluorophenyl)-1*H*-pyrazol-3-yl]methyl}-1-methyl-3-(trifluoromethyl)-1*H*-pyrazol-5-amine
5-(2-chloro-6-fluorophenyl)-N-(2-methyl-4,5,6,7-tetrahydro-2H-indazol-3-yl)-1H-pyrazole-3-carboxamide
and pharmaceutically acceptable salts, N-oxides and isotopically-labeled derivates thereof.

16. A compound as defined in any one of claims 1 to 15, for use in the treatment of the human or animal body by therapy.

17. A compound as defined in any one of claims 1 to 15, for use in the treatment of a pathological condition or disease susceptible to amelioration by inhibiting calcium release-activated calcium channel (CRAC) activity.

18. A compound for use according to claim 17, wherein the pathological condition or disease is selected from an allergic, inflammatory and autoimmune disorders including asthma, chronic obstructive pulmonary disease, bronchiectasis, cystic fibrosis, allergic rhinitis, allergic conjunctivitis, allergic dermatitis, atopic dermatitis, food allergies, seasonal allergies, allergic and inflammatory ophthalmic diseases (such as corneal dystrophy, uveitis, trachoma, sympathetic ophthalmitis), psoriasis, eczema, rheumatoid arthritis, inflammatory bowel disease (such as Barrett's oesophagus, ileitis, ulcerative colitis and Crohns disease), systemic lupus erythematosus, pemphigus vulgaris, multiple sclerosis, thrombosis, polyposis as well as mast cell leukaemia, chronic lymphocytic leaukaemia, B cell lymphoma, breast and prostate cancer, immune thrombocytopenic purpura and macular degeneration, preferably psoriasis, asthma and chronic obstructive pulmonary disease.

19. A pharmaceutical composition comprising a compound as defined in any one of claims 1 to 15 in association with a pharmaceutically acceptable diluent or carrier.

20. Use of a compound as defined in any one of claims 1 to 15, for the manufacture of a medicament for the treatment of a pathological condition or disease as defined in any one of the claims 17 and 18.

21. A method for treating a subject afflicted with a pathological condition or disease as defined in any one of claims 17 to 18, which comprises administering to said subject a therapeutically effective amount of a compound as defined in any one of claims 1 to 15, or a pharmaceutical composition as defined in claim 19.

22. A combination product comprising (i) a compound as defined in any one of claims 1 to 15; and (ii) another compound selected from:
a. Anticholinergics, such as aclidinium bromide or tiotropium,
b. Corticosteroids, or gluococorticoids such as prednisone or methylprednisolone,
c. Antihistamines, such as ebastine, ranitidine, ABT-239 or JNJ 7777120;
d. Beta-2 agonists, such as salmeterol or formoterol,
e. Chemokine receptor antagonists, such as maraviroc or enfuvirtide,
f. CRth2 antagonists,
g. Leukotriene receptor antagonists,
h. JAK inhibitors such as tofacitinib or INCB018424,
i. Syk inhibitors such as R-343,
j. Phosphosdiesterase IV inhibitors such as roflumilast or revamilast,
k. Dual beta-2 adrenoceptor agonist/M3 receptor antagonist (MABA compounds) such as GSK-961081,
l. p38 Inhibitors such as ARRY-797,
m. PKC inhibitors such as NVP-AEB071,
n. 5-lipoxygenase activating protein inhibitors, such as veliflapon,
o. 5-lipoxygenase inhibitors,
p. CYSLTR1 antagonists, such as montelukast, pranlukast or zafirlukast;
q. CYSLTR2 antagonists, such as pranlukast, zafirlukast or tipilukast;
r. BLT1 antagonists,
s. BLT2 antagonists,
t. Thromboxane A2 antagonists such as ramatroban,
u. DP1 receptor antagonists, such as laropiprant,
v. DP1 receptor agonists, such as BW-245C,
w. IP receptor agonists, such as RO-1138452,
x. Anti-IgE, such as omalizumab,
y. IL5 antibody, such as mepolizumab,
z. Leukotriene formation inhibitors,
aa. Bronchodilators, such as pirbuterol, epinephrine, ephedrine or salbutamol;
bb. Decongestants, such as ephedrine, levo-methamphetamine, naphazoline, oxymetazoline, phenylephrine, phenylpropanolamine, propylhexedrine, pseudoephedrine, synephrine or tetrahydrozoline;
cc. Mucolytics such as acetylcysteine, ambroxol, bromhexine, carbocisteine, domiodol, eprazinone, erdosteine, letosteine, neltenexine, sobrerol, stepronin or tiopronin;
dd. Antitussives, such as dextromethorphan,
ee. Analgesics such as aspirin, paracetamol, rofecoxid, celecoxib, morphine, codeine, oxycodone, hydrocodone, dihydromorphine or flupirtine; and
ff. Expectorants such antimony pentasulfide, guaiacolsulfonate, guaifenesin, potassium iodide or tyloxapol.
for simultaneous, separate or sequential use in the treatment of the human or animal body.
